# EUROPÄISCHE PATENTSCHRIFT

(11) **EP 1 409 670 B1**
(45) Veröffentlichungstag und Bekanntmachung des Hinweises auf die Patenterteilung: **13.10.2010**
(21) Anmeldenummer: 02743264.0
(22) Anmeldetag: 05.07.2002
(51) Int. Cl.: C12N 15/113, A61K 31/713, C12Q 1/68

(54) **SYNTHETISCHE DOPPELSTRÄNGIGE OLIGONUCLEOTIDE ZUR GEZIELTEN HEMMUNG DER GENEXPRESSION**
SYNTHETIC DOUBLE STRANDED OLIGONUCLEOTIDES FOR TARGETED INHIBITION OF GENE EXPRESSION
OLIGONUCLEOTIDES SYNTHETIQUES A DOUBLE BRIN UTILISES POUR INHIBER DE FA ON CIBLEE L'EXPRESSION GENIQUE

(30) Priorität: 12.07.2001 DE 10133858
(43) Veröffentlichungstag der Anmeldung: 21.04.2004
(73) Patentinhaber: Sanofi-Aventis Deutschland GmbH, 65929 Frankfurt am Main (DE)
(72) Erfinder: UHLMANN, Eugen, 61479 Glashütten (DE); HUBER, Jochen, 67133 Maxdorf (DE); GUNKEL, Niki, 69121 Heidelberg (DE); NEUMANN, Sandra, 63073 Offenbach (DE)
(86) Internationale Anmeldenummer: PCT/EP2002/007480
(87) Internationale Veröffentlichungsnummer: WO 2003/008576

(56) Entgegenhaltungen:
- WO-A-00/04189
- WO-A-94/01550
- US-A- 5 886 165
- MA M Y-X ET AL: "DESIGN AND SYNTHESIS OF RNA MINIDUPLEXES VIA A SYNTHETIC LINKER APPROACH" BIOCHEMISTRY, AMERICAN CHEMICAL SOCIETY. EASTON, PA, US, Bd. 32, Nr. 7, 1993, Seiten 1751-1758, XP001145414 ISSN: 0006-2960
- ELBASHIR SAYDA M ET AL: "Duplexes of 21-nucleotide RNAs mediate RNA interference in cultured mammalian cells" NATURE, MACMILLAN JOURNALS LTD. LONDON, GB, Bd. 411, Nr. 6836, 24. Mai 2001 (2001-05-24), Seiten 494-498, XP002206451 ISSN: 0028-0836
- DOETSCH P ET AL: "SYNTHESIS AND CHARACTERIZATION OF 2'-5' OLIGO 3' DEOXY ADENYLATE 5' TRI PHOSPHATE AN ANALOG OF 2'-5' OLIGO ADENYLATE 5' TRI PHOSPHATE" NATURE (LONDON), Bd. 291, Nr. 5813, 1981, Seiten 355-358, XP001121988 ISSN: 0028-0836

## Beschreibung

Die vorliegende Erfindung bezieht sich auf neue Oligonucleotid-Derivate, die zumindest teilweise doppelsträngig sind und die an mindestens einem 3'-Ende einen 2'5'-verknüpften Oligonucleotid-Rest aufweisen und deren Verwendung zur gezielten Inhibition der Genexpression.

Die Hemmung der Genexpression mit Hilfe von synthetischen Nucleinsäuren gewinnt zunehmende Bedeutung. Typische Vertreter dieser synthetischen Nucleinsäuren (Oligonucleotide) sind Antisense Oligonucleotide, Ribozyme, DNA-Enzyme und External Guide Sequences (EGS). "Antisense Oligonucleotide" sind kurze einzelsträngige Nucleinsäure-Derivate, die über Watson-Crick Basenpaarung an eine komplementäre Botenribonucleinsäure (mRNA) binden, deren Übersetzung in das entsprechende Protein gehemmt werden soll. In den meisten Fällen entfalten Antisense Oligonucleotide ihre Wirkung nach einem Mechanismus, der von der zellulären Ribonuclease H (RNase H) unterstützt wird, was durch zahlreiche Studien belegt ist. Die in allen Zellen vorkommende RNase H erkennt einen Doppelstrang aus DNA und RNA und schneidet die zum Oligonucleotid komplementäre mRNA durch Hydrolyse einer oder meist mehrerer Phosphodiester-Bindungen. Bekannt ist, wie die Oligonucleotide modifiziert sein müssen, damit eine Aktivierung der RNase H stattfinden kann. Dies ist beispielsweise in "Uhlmann (2000) Curr. Opin. Drug Discov. Dev. 3, 203-213" beschrieben. Synthetische Ribozyme bringen diese Nucleaseaktivität in Ihrer Sequenz mit. Der bekannteste Ribozymtyp sind die sogenannten "Hammerhead" Ribozyme, in welchen die von natürlich vorkommenden Ribozymen abgeleitete Konsensus-Sequenz GAAAC den RNase-Teil bildet und die flankierenden Sequenzen den Antisense Oligonucleotid-Teil bilden. In ähnlicher Weise wirken DNA-Enzyme, die sich allerdings nicht von natürlich vorkommenden Ribozym-Motiven ableiten, sondern durch in vitro Selektion gefunden wurden. EGS sind synthetische RNA-Analoga, welche die zelluläre RNase P aktivieren und über entsprechende flankierende Sequenzen an die Ziel-mRNA binden und einen spezifischen mRNA-Abbau einleiten. Die oben besprochenen Oligonucleotid-Derivate werden alle derart eingesetzt, dass der RNA-Bindungsteil einzelsträngig vorliegt und durch Wechselwirkung mit der Ziel-mRNA die Genexpression in sequenzspezifischer Weise gehemmt wird.

Die Genexpression kann auch durch Wechselwirkung mit bestimmten Proteinen mit Hilfe sogenannter "Decoy"-Oligomere, die die Bindungsregionen für Transkriptionsfaktoren nachahmen, gehemmt werden. Durch die Behandlung mit Decoy-Agenzien lassen sich bestimmte Proteine, insbesondere Transkriptionsfaktoren sequenzspezifisch abfangen und dadurch eine Aktivierung der Transkription verhindern.

Schliesslich gibt es Oligonucleotid-Derivate, die auf der DNA-Ebene wirken. Hierzu zählen Triplex Forming Oligonucleotide (Anti-Gen Oligonucleotide). "Anti-Gen Oligonucleotide" binden über Hoogsteen-Basenpaarung in die große Furche der DNA-Doppelhelix unter Ausbildung einer Tripelhelix, wodurch die Transkription der Gene sequenzspezifisch gehemmt wird. Eine weitere Gruppe von intrazellulär wirkenden Oligonucleotid-Derivaten, die Chimeraplasten, werden zur gezielten Genkorrektur herangezogen.

Ein gemeinsames Problem der Inhibition der Genexpression mit Hilfe synthetischer Oligonucleotide besteht darin, dass stets eine größere Anzahl von Oligonucleotiden gegen verschiedene Bereiche der Ziel-Nucleinsäure getestet werden müssen, um eine effiziente Sequenz zu identifizieren. Weiterhin erfolgt die Inhibition der Genexpression durch Antisense Oligonucleotide oft nur ineffizient oder unvollständig. Zudem wurden sequenz-unspezifische Nebenwirkungen beobachtet, die darauf beruhen können, dass relativ kurze Teilsequenzen von etwa fünf Basen Länge bereits die RNase H aktivieren. Dies wird beispielsweise von "Woolf et al. (1992). Proc. Natl. Acad. Sci. U. S. A. 89, 7305-7309)" gezeigt. Es gibt aber auch Nebeneffekte, die auf der Wechselwirkung der Antisense Oligonucleotide mit Proteinen beruhen.

Kürzlich wurde die Verwendung doppelsträngiger RNA zur Hemmung der Genexpression beschrieben. Doppelsträngige RNA (dsRNA) ist für bestimmte Zellen und Organismen ein Signal, einen sequenzspezifischen Abbau der mRNA nach einem Prozess einzuleiten, der als RNA-Interferenz (RNAi) bekannt ist. Das Phänomen des RNAi wurde in einer Reihe verschiedener Organismen beobachtet, wie beispielsweise C. elegans, Fliegen, Pilzen, Pflanzen und Maus-Embryonen. Man glaubt, dass RNAi dem in Pflanzen beobachten Post-Transcriptional Gene Silencing (PTGS) sehr ähnlich bzw. mit diesem identisch ist. Durch einfache Injektion von dsRNA mit einer Länge von mehr als 500 Basenpaaren (bp), deren Sense-Strang Sequenz mit der zu inhibierenden Ziel-mRNA identisch ist, kann die Expression eines Ziel-Gens mit der entsprechenden DNA-Sequenz spezifisch gehemmt werden. Die Genexpression nicht homologer Gene wird dabei nicht beeinträchtigt. Die Basensequenz des Ziel-Gens wird dabei nicht verändert. RNAi ist ein post-transkriptioneller Prozess, bei dem die dsRNA zuerst in kleinere Fragmente gespalten wird, welche dann wahrscheinlich zum sequenzspezifischen Abbau der Ziel-mRNA Verwendung finden. Neben der Doppelstrang-Nuclease-Aktivität werden auch noch eine ATP-abhängige Helikase-Aktivität diskutiert. Der genaue Mechanismus dieses Vorgangs ist aber nicht bekannt. Untersuchungen in Pflanzen zeigen, dass kleine dsRNA-Fragmente von etwa 25 Nucleotiden Länge die "aktiven Spezies" des RNAi darstellen, welche die sequenzspezifische Erkennung der Ziel RNA auf eine zelluläre Ribonuclease übertragen.

Die Effizienz der Inhibition der Genexpression mit Hilfe von dsRNA nimmt mit abnehmender Fragment-Länge der dsRNA drastisch ab. So wurde gefunden, dass dsRNA von 400 bis 540 bp die Genexpression sehr effektiv hemmt, während 200 bis 300 bp dsRNA weniger effizient hemmt, und dsRNA von 50 bis 100 bp überhaupt nicht mehr wirkt. Erst seit kurzem ist bekannt, dass kleine dsRNA-Fragmente mit einer Länge von 26 bis 81 bp doch einen RNAi-ähnlichen Prozess auszulösen vermögen. Allerdings scheint die dabei beobachtete Inhibition wesentlich schwächer zu sein als bei langen dsRNA-Fragmenten. Die mit dsRNA von 717 bp erzielte Inhibition war deutlicher ausgeprägter als mit dsRNA von kleiner als 200 bp Länge. Die 26 bp dsRNA war ca. 250-mal weniger aktiv als die 81 bp dsRNA. Zudem war die Inhibition sequenzabhängig, da eine andere 27 bp dsRNA überhaupt nicht aktiv war.

In "Elbashir et al. Nature (2001) 411, 494)" wurde eine Inhibition der Genexpression in Zellkultur mit einer 21 Nucleotide umfassenden doppelsträngigen RNA beschrieben. Die entsprechenden dsRNA-Moleküle enthielten an den 3'-Enden in beiden Strängen Überhänge von 2 Nucleotiden, die 3'5'-verknüpft sind, wobei die überhängenden Nucleotide Uracil- oder Thymin-Basen aufweisen. Es wird hierin auch darauf hingewiesen, dass 2'5'-oligoadenylat-aktivierte Ribonucleoase-Prozesse zu einem intrinsischen sequenz-unspezifischen Abbau der Ziel-RNA führen. Ein Nachteil ist aber offensichtlich, dass der Erfolg der Inhibition stark von der verwendeten Zelllinie abhängt.

WO-A-94/01550 offenbart selbst- stabilisierende Oligonukleotide die, durch (teilweise) komplementär zu sein, eine sogenannte 'Hairpin'-Strucktur bilden. Die beiden Stränge des Oligonukleotids sind durch einen Linker verbunden.

Bisher wurde eine effiziente Inhibition der Genexpression hauptsächlich mit dsRNA von über 100 bp Länge erreicht. Diese relativ lange dsRNA ist nur über in vitro oder in vivo Transkription aus der entsprechenden DNA über geeignete Transkriptionssysteme zugänglich ist. Eine andere Limitierung des RNAi mit langer dsRNA besteht darin, dass nur bestimmte Organismen, wie C. elegans, Zebrafisch, Pflanzen, bestimmte Pilzsorten, Drosophila, Oocyten und Embryonen von Mäusen, sequenzspezifische Inhibition mit dsRNA zulassen, während die meisten tierischen Zellen bei Behandlung mit dsRNA Apoptose auslösen. Bei langer dsRNA wird auch noch eine Inhibition der Genexpression beobachtet, wenn die Sequenzhomologie 70 bis 90% beträgt. Daher kann es bei Gen-Familien mit hoher Sequenzhomologie zu Fehlinterpretationen des Phänotyps kommen, indem die Expression mehrerer nicht vollständig homologer Gene gleichzeitig gehemmt wird.

Die Behandlung von Zellen mit dsRNA, wie z. B. mit dsRNA-Viren, führt im allgemeinen zu einem apoptotischen Process bzw. zum sequenzunspezifischen Abbau der mRNA infolge der Induktion einer 2'5'-Oligoadenylat-Synthase-Aktivität. Als Antwort auf die virale dsRNA synthetisiert die infizierte Zelle trimeres oder tetrameres Adenylat (2'5'-A) mit der ungewöhnlichen 2'5'-Phosphodiester Intemucleosidbrücke. Das 2'5'-A wird durch zelluläre Kinasen am 5'-Ende phosphoryliert und aktiviert dann eine Nuclease, die RNase L genannt wird. Man kann das 2'5'-A auch chemisch synthetisieren und der Zelle zuführen (Torrence et al. (1994) Curr. Med. Chem 1, 176-191). Das synthetische 2'5'-A aktiviert die RNase L aber nur, wenn es in das 5'-Phosphat oder 5'-Triphosphat übergeführt wurde. Die durch 5'-p-2'5'-A (p bedeutet Phosphat, Diphosphat oder Triphosphat) aktivierte RNase L baut dann die gesamte RNA der Zelle in sequenzunspezifischer Weise ab. Es wurde auch gezeigt, dass man mit Hilfe von Antisense Oligonucleotid-Konjugaten mit einem 5'-p-2'5'-A-Rest die Genexpression sequenzspezifisch hemmen kann. Dazu ist es aber essentiell, dass das 5'-Ende des 2'5'-A Restes nicht mit dem Oligonucleotid verknüpft ist, sondern als Phosphat oder Triphosphat vorliegt (Torrence et al. (1993) Proc. Natl. Acad. Sci. U. S. A. 90, 1300-4). Weiterhin muss der die Ziel-RNA erkennende Oligonucleotidteil (Antisense-Teil) in einzelsträngiger Form vorliegen. Oligonucleotide mit 2'5'-A Resten an den 3'-Enden, die folglich keine freie 5'-Phosphat- oder Triphosphat-Funktion aufweisen, sind aus oben genannten Gründen bisher nicht zur Inhibition der Genexpression beschrieben worden. Die Hemmung der Genexpression mit den einzelsträngigen 5'-phosphorylierten 5'-p-2'5'-A-Antisense Oligonucleotid-Konjugaten ist eine Variation des Antisense Prinzips und unterliegt somit auch den Limitierungen des Antisense Oligonucleotid-Ansatzes.

In letzter Zeit werden Oligonucleotide in zunehmenden Maße als Werkzeuge zum Studium der Funktion neuer Gene verwendet (Functional Genomics). Der Einsatz von Antisense Oligonucleotiden und Ribozymen zur sequenzspezifischen Inhibition der Genexpression neuer Gene, die Proteine mit unbekannter Funktion kodieren, wird dadurch erschwert, dass im allgemeinen viele verschiedene Oligonucleotide unterschiedlicher Sequenzen getestet werden müssen, was besonders im großen Durchsatz hinderlich ist.

Die Aufgabe dieser Erfindung bestand also darin, neue chemisch modifizierte Oligonucleotide mit signifikant verbesserter Hemmung der Genexpression zur Verfügung zu stellen, welche die oben genannten Limitierungen der herkömmlichen Methoden und Agenzien umgehen.

Die Aufgabe wurde erfindungsgemäß gelöst durch neue Oligonucleotid-Derivate, die zumindest teilweise doppelsträngig sind und die an mindestens einem 3'-Ende einen 2'5'-verknüpften Oligonucleotid-Rest aufweisen. Die Sequenz der neuen Oligonucleotid-Derivate ist in einem Strang komplementär zur RNA-Sequenz, deren Translation gehemmt werden soll, im anderen Strang entspricht sie der zu hemmenden RNA. Der RNA-Doppelstrang entspricht somit der Basensequenz des Gens, dessen Expression gehemmt werden kann, wobei für die Desoxyribonucleotide die entsprechenden Ribonucleotide und Uridin für Thymidin stehen.

Die Erfindung betrifft daher doppelsträngige Nucleinsäure-Derivate der Formel I, 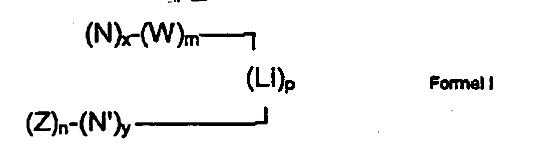 wobei
N und N' natürlich oder nicht natürlich vorkommende Nucleotide sind, von denen 21 Nukleotide zueinander komplementär sind und wobei der Nucleotidstrang (N')_{y} der nicht-kodierende Strang, bzw. der zum Ziel-Gen komplementäre Strang ist,
x und y gleich je 21 sind,
n unabhängig gleich 4 bis 6 ist,
m unabhängig gleich 0 bis 20 ist,
p unabhängig gleich 0 bis 10 ist,
W und Z natürlich oder nicht natürlich vorkommende Nucleotide sind, die über eine 3'5'- oder 2'5'-Internucleosid-Bindung verknüpft sind,
Li ein Linker ist, der die beiden Nucleotidstränge kovalent verknüpft,
**dadurch gekennzeichnet, dass** mindestens zwei Reste Z und gegebenenfalls zwei Reste W über eine 2'5'-Intemueleosid-Bindung verknüpft sind und einzelsträngig vorliegen.

Bevorzugt sind Oligonucleotide der Formel I, deren homologe Ziel-RNA folgende Sequenzmuster aufweist:

5'-(U)ᵥ-(N)_{z}-(U)_{w}

5'-(U)ᵥ-(N)_{z}-UX

5'-UX-(N)_{z}-UX und

5'-(U)ᵥ-(N)_{z}

wobei v und w unabhängig voneinander gleich 2 bis 20, bevorzugt 2 bis 10, besonders bevorzugt 2 bis 6 sind und
z gleich 15 bis 25, bevorzugt 16 bis 23 und besonders bevorzugt 19 bis 21 ist und U gleich Uridin, N gleich A, G, C oder U, und X gleich A, G oder C, bevorzugt A, sind.

Enthält das Gen, dessen Expression gehemmt werden soil, beispielsweise folgende DNA-Sequenz
5'-TTTTGAAGCGAAGGTTGTGGATCTG (Seq ID Nr. 1)
bzw. folgende RNA-Sequenz
5'-UUUUGAAGCGAAGGUUGUGGAUCUG (Seq ID Nr. 2)
dann hat die Ziel-RNA folgendes Sequenzmuster
5'-(U)ᵥ-(N)_{z}-UX, wobei v gleich 4, z gleich 19 und X gleich G sind.

Weiterhin bevorzugt sind Oligonucleotide der Formel I, in denen eine oder mehrere Phosphodiester-Bindungen ersetzt sind, beispielsweise durch Phosphorothioat-Bindungen oder N3',P5'-Phosphoramidat-Bindungen . Besonders bevorzugt sind Oligonucleotide der Formel I, in denen eine oder mehrere Phosphodiester-Bindungen durch Phosphorothioat-Reste ersetzt sind. Die Einführung der Phosphorothioat-Reste erfolgt vorzugsweise an den 3'-Enden, den 5'-Enden sowie an den internen Pyrimidin-Nucleotiden C und U, insbesondere dann, wenn mehrere Pyrimidin-Nucleotide in der Sequenz aufeinander folgen. Die Einführung der Phosphorothioat-Reste kann im oberen oder unteren Strang erfolgen, vorzugsweise erfolgt sie in beiden Strängen.

Eine besondere Ausführungsform der Erfindung besteht darin, dass zwei oder mehrere Oligonucleotid-Derivate gemäss Fomel I als Gemisch zur Inhibition der Genexpression eingesetzt werden. Dabei können die Oligonucleotid-Derivate gegen unterschiedliche Regionen einer RNA oder gegen die RNA unterschiedlicher Gene gerichtet sein.
Überraschenderweise wurde gefunden, dass partiell doppelsträngige Nucleinsäuren, die an mindestens einem Ende einen 2'5'-verknüpften Oligoadenylat-Rest aufweisen, eine viel stärkere Inhibition der Genexpression bewirken als doppelsträngige RNA, die nur aus 3'5'-verknüpften Nucleotiden besteht. Die doppelsträngigen RNA-Fragmente mit dem 2'5'-verknüpften Oligoadenylat-Rest waren auch aktiver als der entsprechende Einzelstrang mit einem 2'5'-verknüpften Oligoadenylat-Rest. Dies ist überraschend, da sich normalerweise Antisense Oligonucleotid und Sense Oligonucleotid in der Wirkung aufheben. Überraschend ist auch, dass der 2'5'-verknüpfte Oligoadenylat-Rest kein freies Ende mit einem 5'-Phosphat- oder 5'-Triphosphat-Rest haben muss, um seine Aktivität entfalten zu können. Es war auch völlig überraschend, dass der 2'5'-verknüpfte Oligoadenylat-Rest über die 5'-Funktion direkt an die 3'5'-verknüpfte RNA gebunden sein kann. Ein 2'5'-A Rest im codierenden Strang hatte überraschenderweise nur einen sehr geringen positiven Einfluss auf die Aktivität des Doppelstrangs, sofern sich im nicht-codierenden Strang ein 2'5'-A Rest befand. Überraschenderweise ist doppelsträngige RNA mit vier bis sechs Basen überhängenden Enden am unteren Strang viel aktiver als solche mit nur zwei überhängenden Basen. Im Gegensatz zu den Antisense Oligonucleotiden, bei denen im allgemeinen mehrere (beispielsweise 10 bis 100) Sequenzen getestet werden müssen, um eine aktive Sequenz zu erhalten, waren überraschenderweise alle getesteten doppelsträngigen Oligonucleotide der Formel I inhibitorisch aktiv, wenn diese zu den entsprechenden Gensequenzen homolog waren. Überraschenderweise wurde auch keine intrinsische Unspezifität mit den 2'5'-verknüpften Oligonucleotiden beobachtet. Bisher hatte man eine 2'5'-aktivierte Inhibition über doppelsträngige RNA stets mit einem unspezifischen, also sequenzunabhängigen, Effekt in Zusammenhang gebracht (Bass, Nature (2001) 411, 428).

Zwei 2'5'-(A)₄- Oligonucleotide, die nicht basenpaaren, sind im Gemisch im Vergleich zu den dopplesträngig vorliegenden Molekülen weniger wirksam. Weiterhin sind Verbindungen der Formel I weniger oder unwirksam, wenn die Sequenzhomologie zur Ziel-RNA nicht perfekt ist.

Überraschenderweise waren die erfindungsgemässen Oligonucleotide auch in primären humanen Zellen inhibitorisch in sequenzspezifischer Weise wirksam. Unseres Wissens wurde die Inhibition der Genexpression durch doppelsträngige Oligonucleotide bislang noch nicht in primären menschlichen Zellen beobachtet. Es war ebenfalls unerwartet, dass hierzu nur ein Strang der doppelsträngigen RNA ein überhängendes Ende aufweisen musste.

Die erfindungsgemässen Oligonucleotide der Formel I können auch zur Hemmung der Genexpression in Zellen, die nur wenig, keine oder defekte 2'5'-Oligoadenylat-Synthase exprimieren, eingesetzt werden. Die beschriebenen 21 Nucleotide umfassenden dsRNA-Moleküle (Elbashir et al. Nature (2001) 411, 494) besitzen diese Eigenschaften nicht.

Weiterhin können die Oligonucleotide der Formel I auch zur Behandlung von Patienten mit einer Defizienz oder einem Defekt der 2'5'-Oligoadenylat-Synthase eingesetzt werden. Beispielsweise können auch Patienten mit CFS (Chronic Fatigue Syndrome) behandelt werden.

Die Auswahl der Sequenzen der doppelsträngigen Nucleinsäuren, die zur Inhibition der Genexpression bestimmter Targets eingesetzt werden, erfolgt anhand der entsprechenden Gen-Sequenzen. Die Sequenzen dieser Gene werden durch Sequenzierung oder aus Gen-Datenbanken erhalten. Als Beispiel sei hier die Inhibition der Luciferase (firefly) mit doppelsträngigen Nucleinsäuren erläutert. Die Zugangsnummer für dieses Gen ist U47298. Der codierende Bereich der Firefly Luciferase umfasst 1653 Nucleotide. Es können beispielsweise unter anderem die folgenden vier Bereiche als Zielsequenzen für die Inhibition mit doppelsträngigen Nucleinsäuren ausgewählt werden.

Die entsprechende doppelsträngige RNA für diese Bereiche hat dann folgende Sequenz.

Die davon abgeleiteten erfindungsgemäßen doppelsträngen Nucleinsäuren haben beispielsweise nachfolgend aufgeführte Sequenzen und sind dadurch charakterisiert, dass in mindestens einem Strang nämlich dem nicht codierenden Strang, zwei oder mehr Nucleotide (hier mit kleinen Buchstaben gekennzeichnet) durch eine 2'5'-Internucleosid-Bindung verknüpft sind. Die überhängenden Enden müssen nicht komplementär zur Ziel RNA sein. Die Ziffer gibt die Region auf der RNA wieder, up bedeutet oberer (codierender) Strang und Io bedeutet unterer (nicht codierender) Strang. Bevorzugt sind 2'5'-verknüpfte Adenylat-Reste. Wenn in Formel I p gleich null ist, werden die beiden Stränge nur über Wasserstoffbrücken zusammengehalten.

Die Oligonucleotide können aber auch im unteren Strang einen Überhang von beispielsweise 6 Nucleotiden aufweisen, wobei diese partiell oder alle 2'5'-verknüpft sind.

Ein Strang des doppelsträngigen Oligonucleotids hat beispielsweise folgende Struktur:

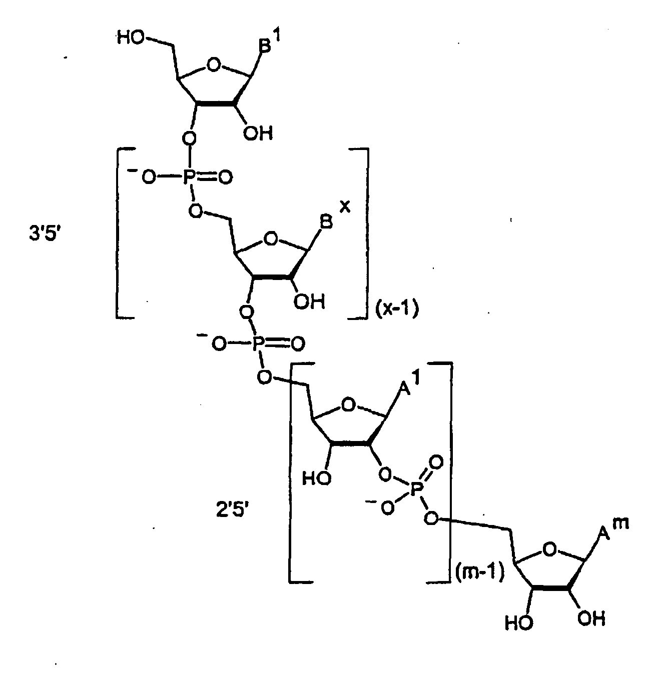

Zur Prüfung auf biologische Aktivität können folgende Oligonucleotide hergestellt, gegebenenfalls zum Doppelstrang hybridisiert und auf Inhibition der Luciferase-Aktivität in Test-Ansätzen geprüft werden. Um zu zeigen, dass die erfindungsgemässen Oligonucleotide auch in Zellen anderer Spezies insbesondere in primären menschlichen Zellen zur Inhibition der Genexpression eingesetzt werden können, kann eine erfindungsgemäße Verbindung beispielsweise gegen ein menschliches Gen bzw. die zugehörige RNA gerichtet und in menschlichen Zellen (HUVEC, human umbilical vein endothelial cells) getestet werden. Hierzu könnte beispielsweise die Edg-1 DNA (Zugangsnummer M31210) aus der Gendatenbank in die entsprechende doppelsträngige RNA umgeschrieben und folgende zwei Bereiche (175 und 725) zur Synthese entsprechender Oligonucleotide ausgewählt werden.

Die entsprechenden Oligonucleotide hätten beispielsweise einen wie folgt offenbarten Aufbau:

Die Ziffer gibt die Region auf der edg-1 RNA wieder, up bedeutet oberer (codierender) Strang und lo bedeutet unterer (nicht codierender) Strang. Die Mismatch Kontrolle bildet in sich einen vollständig gepaarten Doppelstrang, stimmt aber in 5 Nucleotiden (als mismatch unterstrichen) nicht mit der edg-1 RNA überein. FITC ist ein kommerziell erhältlicher Fluoreszenzmarker.

Weiterhin wurden folgende gegen edg-1 gerichtete Oligonucleotide mit verbesserter Nucleasestabilität und erhöhter inhibitorischer Aktivität hergestellt, die sich von den vorherigen edg-1 Sequenzen ableiten.

In einer anderen Ausgestaltung der Erfindung werden doppelsträngige Oligonucleotide eingesetzt, die nur in einem Strang, dem nichtcodierenden Strang, einen 2'5'-verknüpften überhängenden Rest aufweisen, wobei die beiden Stränge über eine oder mehrere kovalente Bindungen zusammen gehalten werden. Diese kovalente Bindung kann beispielsweise ein Linker vom Typ (Li)p sein.

Beispielsweise können die beiden Nucleinsäure-Stränge über mehrere Nucleotid-Reste, bevorzugt vier bis fünf Nucleotid-Reste (Li gleich N, bevorzugt Thymidin, p gleich 4 bis 20, bevorzugt 4 oder 5), zusammengehalten werden.

Im Falle der abasischen Linker hat das Molekül beispielsweise folgende Formel:

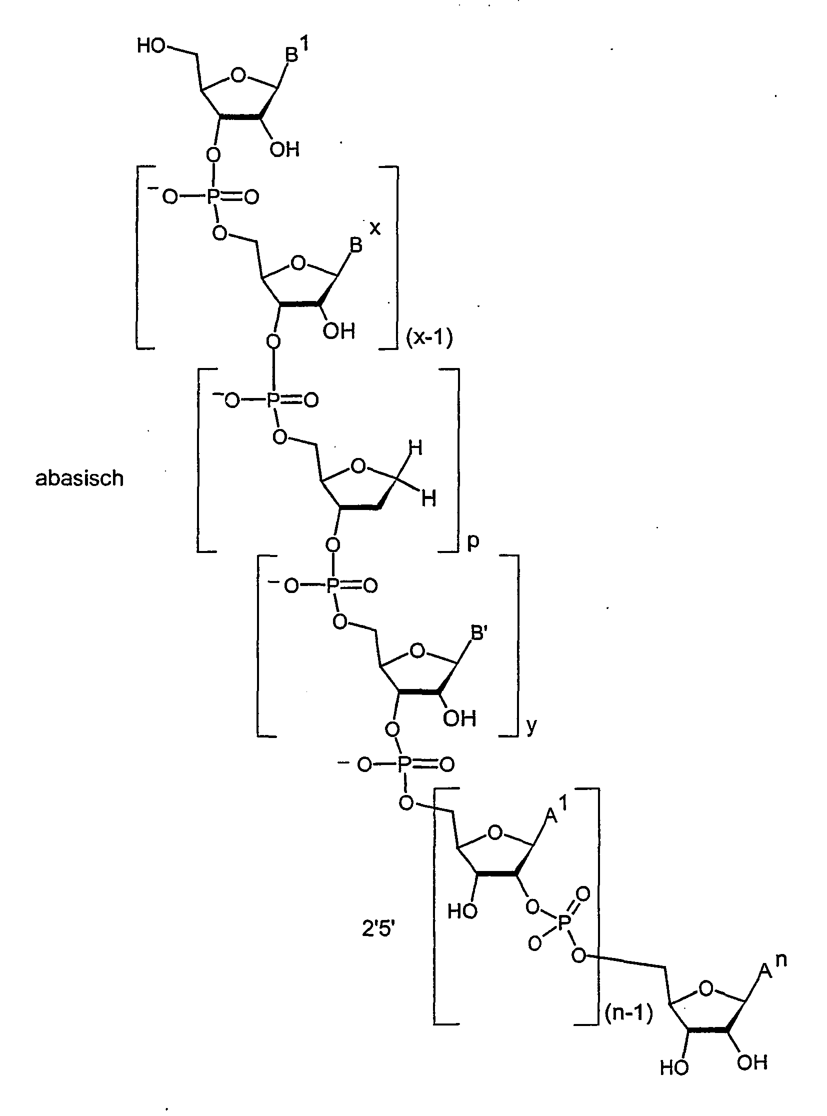

Die beiden Stränge können aber auch über nicht-nucleotidische Reste zusammengehalten werden. Als nicht-nucleotidische Linker kommen beispielsweise ein oder mehrere Oligoethylenglycol-Phosphatreste infrage, bevorzugt Tri- und Hexaethylenglycol-Phosphatreste. Andere Linker sind beispielsweise Alkan-diol-Phosphate, bevorzugt Propan-1.3-diol-Phosphat, Butan-1.4-diol-Phosphat und Dodecyl-1.12-diol-Phosphat. Weitere Linker sind beispielsweise die abasischen 1'.2'-Dideoxyribose-Einheiten, die im allgemeinen 3'-O, 5'-O-verknüpft werden. Die für die Synthese notwendigen Linker-Reagenzien sind größtenteils kommerziell erhältlich (z.B. Spacer 9, Spacer 18, Spacer C3, Spacer C12, dSpacer (abasisch) der Firma Glen Research, Sterling, VA). In den Linker können auch funktionelle Gruppen eingebaut werden, welche beispielsweise die zelluläre Aufnahme des doppelsträngigen Oligonucleotids erhöhen, die Bioverfügbarkeit erhöhen; die Nucleasestabilität erhöhen, oder die biologische Aktivität steigern. Weiterhin lassen sich im Linker Gruppen zur Markierung, beispielsweise Fluoreszenz-Marker oder Biotin-Marker einbauen.

Andere Linker zur Verknüpfung der beiden Stränge sind beispielsweise DisulfidBrücken (-S-S-) oder Pyrothiophosphat-Brücken (-(O₃)-P-S-S-P(O₃)-).

Alternativ können die Doppelstränge nichtkovalent über lipophile oder ionische Wechselwirkungen oder Wasserstoffbrücken zusammen gehalten werden.

Die Spezifität der Inhibition der Luciferase-Expression wurde anhand von doppelsträngigen Kontroll-Oligonucleotiden überprüft, die nicht vollständig homolog zur Ziel-RNA sind und beispielsweise 2 oder 4 Basen-Mißpaarungen aufweisen. Andere Kontroll-Oligonucleotide haben eine Variation bezüglich der überhängenden Enden.

Folgende Oligonucleotide wurden hergestellt, die beispielsweise im oberen, im unteren oder in beiden Strängen, entweder als Phosphorothioat (Sternchen) oder als 2'O-Methylribonucleotid (unterstrichen) modifiziert sind.

Die erfindungsgemässen Nucleinsäure-Derivate der Formel I sind aus Oligonucleotiden aufgebaut. Ein Oligonucleotid kann beispielsweise vollständig aus den Nukleotiden Adenosinphosphat, Guanosinphosphat, Inosinphosphat, Cytidinphosphat, Uridinphosphat und Thymidinphosphat aufgebaut sein. Bevorzugt sind Oligonucleotide, die aus Ribonucleotiden aufgebaut sind, die sogenannten Oligoribonucleotide. In anderen Ausführungsformen der Erfindung kann ein Oligonucleotid gegebenenfalls ein oder mehrere Modifikationen, beispielsweise chemische Modifikationen, enthalten. Ein Oligonucleotid kann mehrere gleiche und/oder verschiedene Modifikationen aufweisen.

Beispiele für chemische Modifikationen sind dem Fachmann bekannt und beispielsweise in E. Uhlmann and A. Peyman, Chemical Reviews 90 (1990) 543 und "Protocols for Oligonucleotides and Analogs" Synthesis and Properties & Synthesis and Analytical Techniques, S. Agrawal, Ed, Humana Press, Totowa, USA 1993, J. Hunziker und C. Leumann 'Nucleic Acid Analogs: Sythesis and Properties' in Modern Synthetic Methods (Ed. Beat Ernst und C. Leumann) Verlag Helvetica Chimica Acata, Basel, S 331-417, RP lyer et al. Curr Opin Mol Therap (1999) 1:344-358; S. Verma und F. Eckstein , Annu Rev Biochem (1998) 67:99-134; JW Engels und E. Uhlmann: Chemistry of oligonucleotides. In: Pharmaceutical aspects of oligonucleotides. Couvreur P, Malvy C (Eds), Taylor & Francis, London, (2000): 35-78, beschrieben.

Die chemische Modifikation eines Oligonucleotids kann beispielsweise
a) den vollständigen oder teilweisen Ersatz der Phosphorsäurediesterbrücken, beispielsweise durch Phosphorothioat-, Phoshorodithioat-, NR¹R^{1'}-Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, (C₁-C₈)Alkylphosphonat- und/oder (C₆-C₁₂)-Arylphosphonat-Brücken, wobei
   R¹ und R^{1'} unabhängig voneinander für Wasserstoff, (C₁-C₁₈)-Alkyl, (C₆-C₂₀)-Aryl, (C₆-C₁₄)-Aryl-(C₁-C₈)-alkyl, bevorzugt für Wasserstoff, (C₁-C₈)-Alkyl und/oder Methoxyethyl, besonders bevorzugt für Wasserstoff, (C₁-C₄)-Alkyl und/oder Methoxyethyl stehen
   oder
   R¹ und R^{1'} zusammen mit dem sie tragenden Stickstoffatom einen 5-6-gliedrigen heterocyclischen Ring bilden, der zusätzlich ein weiteres Heteroatom aus der Reihe O, S, N enthalten kann;
b) den vollständigen oder teilweisen Ersatz der 3'- und/oder 5'-Phosphorsäurediesterbrücken durch "Dephospho"-Brücken (beschrieben beispielsweise in Uhlmann, E. und Peyman, A. in "Methods in Molecular Biology", Vol. 20, "Protocols for Oligonucleotides and Analogs", S. Agrawal, Ed., Humana Press, Totowa 1993, Chapter 16, 355ff), beispielsweise durch Formacetal, 3'-Thioformacetal, Methylhydroxylamin, Oxim, Methylendimethylhydrazo, Dimethylensulfon und/oder Silylgruppen;
c) den teilweisen Ersatz des Zuckerphosphat-Rückgrats, beispielsweise durch "Morpholino"-Oligomere (beispielweise in E. P. Stirchak et al., Nucleic Acids Res. 17 (1989) 6129 und in J. Summerton and D. Weller, Antisense and Nucleic Acid Drug Dev. 7 (1997) 187-195 beschrieben) und/oder durch Polyamid Nucleinsäuren ("PNAs") (beispielsweise beschrieben in P. E. Nielsen et al, Bioconj. Chem. 5 (1994) 3) und/oder Phosphomonosäureester Nukleinsäuren ("PHONAs") (beschrieben beispielsweise in Peyman et al., Angew. Chem. Int. Ed. Engl. 35 (1996) 2632-2638);
d) den teilweisen Ersatz der β-D-Riboseeinheiten, beispielsweise durch β-D-2'-Desoxyribose, α-D-2'-Desoxyribose, L-2'-Desoxyribose, 2'-F-2'-Desoxyribose, 2'-F-2'-Desoxyarabinofuranose, 2'-O-(C₁-C₆)Alkyl-Ribose, 2'-O-(C₂-C₆)Alkenyl-Ribose,2'-[O-(C₁-C₆)Alkyl-O-(C₁-C₆)Alkyl]-Ribose, 2'-NH₂-2'-desoxyribose, β-D-Xylofuranose, β-D-Arabinofuranose, α-Arabinofuranose, 2,4-Dideoxy-β-D-erythro-hexo-pyranose, konformativ eingeschränkte Zuckeranaloga wie LNA (Locked nucleic acids; Singh et al., Chem. Commun. 4 (1998) 455; Singh et al. Chem. Commun. 12 (1998) 1247) und carbocyclische (beschreiben beispielsweise in Froehler, J.Am.Chem.Soc. 114 (1992) 8320) und/oder offenkettige Zuckeranaloga (beschrieben beispielsweise in Vandendriessche et al., Tetrahedron 49 (1993) 7223) und/oder Bicyclo-Zuckeranaloga (beschrieben beispielsweise in M. Tarkov et al., Helv. Chim. Acta 76 (1993) 481). Die 2'-modifizierten Oligonucleotid-Analoga sind in Manoharan, Biochim. Biophys. Acta (1999) 117 und konformativ eingeschränkte Oligonucleotid-Analoga sind in Herdewijn, Biochim. Biopyhs. Acta (1999) 167 detailliert beschrieben.
e) die Modifikation beziehungsweise den vollständigen oder teilweisen Ersatz der natürlichen Nucleosid-Basen, beispielsweise durch 5-(Hydroxymethyl)uracil, 5-Aminouracil, Pseudouracil, Pseudoisocytosin, Dihydrouracil, 5-(C₁-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin oder 7-Deaza-7-substituierte Purine beinhalten. Heterozyklische Basenmodifikationen sind beispielsweise in Herdewijn, Antisense & Nucl. Acid Drug Dev. (2000) 297 beschrieben.

Die chemische Modifikation des Oligonucleotids umfaßt weiterhin die Konjugation eines Oligonucleotids mit einem oder mehreren Molekülen, welche die Eigenschaften (beispielsweise Nucleasestablilität, Affinität zur Target-Sequenz, Pharmakokinetik) des Oligonucleotids günstig beeinflußen und/oder bei der Hybridisierung des modifizierten Oligonucleotids an die Target-Sequenz diese unter Bindung und/oder Quervernetzung angreift (Oligonucleotid-Konjugate). Beispiele dafür sind Konjugate mit Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie (C₁₂-C₂₀)-Alkyl, mit Lipiden wie 1,2-Dihexadecyl-rac-glycerin, mit Steroiden wie Cholesterin oder Testosteron, mit Vitaminen wie Vitamin E, mit Poly- bzw. Oligo-ethylengylcol, mit (C₁₂-C₁₈)-Alkyl-Phosphatdiestem und/oder mit -O-CH₂-CH(OH)-O-(C₁₂-C₁₈)-Alkyl. Solche Moleküle können am 5'- und/oder am 3'-Ende und/oder innerhalb der Sequenz, z.B. an eine Nucleobase konjugiert sein. Dem Fachmann bekannte Oligonucleotid-Konjugate sind beispielsweise in Manoharan (2001) Conjugated Oligonucleotides in Antisense technology. In: Crooke (Herausgeber) Antisense Technology. Marcel Dekker, Nwe York, beschrieben.

Eine spezielle Ausführungsform der chemischen Modifikation betrifft die Konjugation: des Oligonucleotids a) mit lipophilen Molekülen, beispielsweise (C₁₂-C₂₀)-Alkyl, b) mit Steroiden wie Cholesterin und/oder Testosteron, c) mit Poly- und/oder Oligoethylenglykol, d) mit Vitamin E, e) mit Interkalatoren wie Pyren, f) mit (C₁₄-C₁₈)-Alkyl-Phosphatdiestern und/oder g) mit -O-CH₂-CH(OH)-O-(C₁₂-C₁₆)-Alkyl.

Eine weitere spezielle Ausführungsform der chemischen Modifikation betrifft die Derivatisierung des Oligonucleotids wie in HMR 99/L045 beschrieben als Arylester-Konjugat, beispielsweise als FDA-Konjugat, wodurch die zelluläre Aufnahme der Oligonucleotide begünstigt wird.

Verfahren zur Herstellung der Oligonucleotid-Derivate sind dem Fachmann bekannt und z.B. in Uhlmann, E. & Peyman, A., Chem. Rev. 90 (1990) 543 und/oder M. Manoharan in "Antisense Research and Applications", Crooke and Lebleu, Eds., CRC Press, Boca Raton, 1993, Chapter 17, S.303ff. und/oder EP-A 0 552 766 beschrieben. Der Doppelstrang kann beispielsweise durch eine Hybridisierung der beiden Einzelstränge hergestellt werden, indem Lösungen der beiden Einzelstränge in verdünntem Puffer erhitzt und wieder abgekühlt werden. Im Prinzip lassen sich die für die Gensynthese beschriebenen Verfahren zur Herstellung des Dopplestranges einsetzen (Chemical and Enzymatic Synthesis of Gene Fragments (Herausgeber: Gassen und Lang)Verlag Chemie, Weinheim (1982).

In weiteren speziellen Ausführungsformen der Erfindung kann das Oligonucleotid an einem 3' und/oder am 5'-Ende 3'-3'- und/oder 5'-5'-Inversionen aufweisen. Diese Art der chemischen Modifikation ist dem Fachmann bekannt und beispielsweise in M. Koga et al., J. Org. Chem. 56 (1991) 3757 beschrieben.

Der 2'5'-überhängende Rest kann beispielsweise Adenosin, 3'-Deoxyadenosin (Cordycepin), Inosin, 8-Bromadenosin, 8-Methyladenosin und andere 8-substituierte Adenosin-Derivaten enthalten. Der Ribose-Rest kann auch als 3'-O-Methyladenosin derivatisiert sein. Die Internucleosidbindungen im 2'5'-überhängenden Teil sind bevorzugt Phosphodiester- und Phosphorothioatbindungen. Gängige Derivate des 2'5'-Adenylats, deren Synthese und Aktivierung der RNase L, sind in der Lituratur beschrieben (Player et al. (1998) Pharmacol. Ther. 78, 55).

Die Erfindung betrifft ferner Verfahren zur Herstellung der Oligonucleotide. Die beschriebenen Oligonucleotide können mit Hilfe verschiedener bekannter, chemischer Verfahren, z.B. wie in Eckstein, F. (1991) "Oligonucleotides and Analogues, A Practical Approach", IRL Press, Oxford beschrieben, hergestellt werden. Die Oligonucleotide können auch durch Verfahren hergestellt werden, die gegebenenfalls einen oder mehrere enzymatische Schritte enthalten.

Die Erfindung betrifft weiterhin die Verwendung der Oligonucleotide zur Modulation sowie zur ganzen oder teilweisen Inhibition der Expression von bestimmten Target-Genen, beispielsweise zur ganzen oder teilweisen Inhibition der Translation. Die Erfindung betrifft weiterhin die Verwendung der Oligonucleotide zur Modulation sowie zur ganzen oder teilweisen Inhibition der Expression in Zellen, die nur wenig, keine oder defekte 2'5'-Oligoadenylat-Synthase aufweisen.

Die Erfindung betrifft weiterhin die Verwendung der Oligonucleotide als Arzneimittel bzw. die Verwendung der Oligonucleotide zur Herstellung von Arzneimitteln. Insbesondere können die Oligonucleotide in Arzneimitteln, die zur Prävention und/oder Behandlung von Krankheiten, die mit der Expression bzw. einer Überexpression bestimmter Gene einhergehen, eingesetzt werden.

Die Erfindung betrifft weiterhin die Verwendung der Oligonucleotide bzw. von Arzneimitteln, die diese Oligonucleotide enthalten, zur Behandlung von Krankheiten, bei denen definierte Gene durch Überexpression ursächlich bzw. beteiligt ist.

Die Arzneimittel der vorliegenden Erfindung können beispielsweise zur Behandlung von Erkrankungen, die durch Viren hervorgerufen werden, beispielsweise durch CMV, HIV, HSV-1, HSV-2, Hepatitis B, Hepatitis C, oder Papilloma Viren, verwendet werden. Arzneimittel dieser Erfindung eignen sich besonders zur Behandlung von RNA Viren wie etwa Polioviren, VSV oder Influenza, insbesondere auch von doppelsträngigen RNA-Viren wie etwa Reoviren.

Die Arzneimittel der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für Krebsentstehung bzw. Krebswachstum verantwortlich sind. Solche Targets sind beispielsweise:
1) Nucleäre Onkoproteine wie beispielsweise c-myc, N-myc, c-myb, c-fos, c-fos/jun, PCNA, p120
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl, c-ets
3) Zelluläre Rezeptoren wie beispielsweise EGF-Rezeptor, Her-2, c-erbA, VEGF-Rezeptor (KDR-1), Retinoid-Rezeptoren, Protein-Kinase regulatorische Untereinheit, c-fms, Tie-2, c-raf-1 Kinase, PKC-alpha, Protein Kinase A (R1 alpha)
4) Cytokine, Wachstumsfaktoren, Extrazelluläre Matrix wie beispielsweise CSF-1, IL-6, IL-1a, IL-1b, IL-2, IL-4, IL-6, IL-8, bFGF, VEGF, Myeloblastin, Fibronectin.
5) Inhibitoren von Tumor Suppressor Genen wie beispielsweise MDM-2.

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Die Arzneimittel der vorliegenden Erfindung eignen sich beispielsweise auch zur Verhinderung der Restenose. Beispielsweise können dabei Oligonucleotid-Sequenzen zum Einsatz kommen, die gegen Targets gerichtet sind, die für Proliferation oder Migration verantwortlich sind. Solche Targets sind beispielsweise:
1) Nucleäre Transaktivator-Proteine und Cycline wie beispielsweise c-myc, c-myb, c-fos, c-fos/jun, Cycline und cdc2-Kinase
2) Mitogene oder Wachstumsfaktoren wie beispielsweise PDGF, bFGF, VEGF, EGF, HB-EGF und TGF-β.
3) Zelluläre Rezeptoren wie beispielsweise bFGF-Rezeptor, EGF-Rezeptor und PDGF-Rezeptor.

Die Erfindung betrifft ferner Oligonucleotide zur Behandlung von Asthma, wobei die Expression des Adenosin-A1-Rezeptors, Adenosin-A3-Rezeptors, Bradikinin-Rezeptors oder von IL-13 mit Hilfe geeigneter Oligonucleotide inhibiert werden.

Die Erfindung betrifft beispielsweise auch Oligonucleotide zur Behandlung von Herz-Kreislauf-Krankheiten, wobei zum Beispiel die Expression des β1-adrenergen Rezeptors oder eines Proteins aus der EDG-Familie, wie beispielsweise Edg-1, gehemmt wird.

Die Erfindung betrifft beispielsweise auch Oligonucleotide zur Behandlung von Diabetes, wobei zum Beispiel die Expression von PTP-1 B gehemmt wird.

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägem für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Bevorzugte Verabreichungsformen sind topische Applikationen, lokale Applikationen wie beispielsweise mit Hilfe eines Katheters oder durch Inhalation, Injektionen bzw. Infusionen und die orale Verabreichung. Zur Injektion werden die Oligonucleotid-Derivate in einer flüssigen Lösung, vorzugsweise in einem physiologisch annehmbaren Puffer, wie z.B. Hank's Lösung oder Ringer's Lösung, formuliert. Die Oligonucleotide können aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.

Die Erfindung betrifft weiterhin pharmazeutische Zubereitungen, die Oligonucleotide und/oder deren physiologisch verträgliche Salze neben pharmazeutisch einwandfreien Träger- und/oder Zusatzstoffen enthalten.

Die Oligonucleotide und/oder deren physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine topische, perkutane, parenterale oder enterale Anwendung gestatten und die als aktiven Bestandteil eine wirksame Dosis mindestens eines Oligonucleotids, neben üblichen pharmazeutisch einwandfreien Träger- und Zusatzstoffen enthalten. Die Zubereitungen enthalten normalerweise etwa 0,1 bis 90 Gew.-% der therapeutisch wirksamen Verbindung. Zur Behandlung von Hautkrankheiten wie beispielsweise Psoriasis oder Vitiligo wird eine topische Anwendung, z.B. in Form von Salben, Lotionen oder Tinkturen, Emulsionen, Suspensionen bevorzugt.

Die Herstellung der pharmazeutischen Präparate erfolgt in an sich bekannter Weise, (z. B. Remingtons Pharmaceutical Sciences, Mack Publ. Co., Easton, PA.), wobei pharmazeutisch inerte anorganische und/oder organische Trägerstoffe verwendet werden. Für die Herstellung von Pillen, Tabletten, Dragees und Hartgelatinekapseln kann man z.B. Lactose, Maisstärke und/oder Derivate derselben, Talk, Stearinsäure und/oder deren Salze etc. verwenden. Trägerstoffe für Weichgelatinekapseln und/oder Suppositorien sind z.B. Fette, Wachse, halbfeste und flüssige Polyole, natürliche und/oder gehärtete Öle etc. Als Trägerstoffe für die Herstellung von Lösungen und/oder Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate und/oder Rods eignen sich Mischpolymerisate aus Glykolsäure und Milchsäure. Darüber hinaus sind Liposomenformulierungen, die dem Fachmann bekannt sind, geeignet (N. Weiner, Drug Develop Ind Pharm 15 (1989) 1523; "Liposome Dermatics, Springer Verlag 1992), beispielsweise HVJ-Liposomen (Hayashi, Gene Therapy 3 (1996) 878). Die dermale Applikation kann beispielsweise auch auch unter Zuhilfenahme ionophoretischer oder Methoden und/oder mit Hilfe der Elektroporation erfolgen. Darüber hinaus können Lipofektine und andere Carriersysteme, beispielsweise solche, die in der Gentherapie Anwendung finden, verwendet werden. Insbesondere sind Systeme geeignet, mit deren Hilfe Oligonucleotide mit großer Effizienz in eukaryotische Zellen eingebracht werden können.

Eine pharmazeutische Zubereitung kann neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel und/oder Lösungsvermittler und/oder Mittel zur Erzielung eines Depoteffekts, sowie Salze zur Veränderung des osmotischen Drucks, Überzugsmittel und/oder Antioxidantien enthalten. Sie können auch zwei oder mehrere verschiedene Oligonucleotide und/oder deren physiologisch verträgliche Salze enthalten sowie ferner neben mindestens einem Oligonucleotid einen oder mehrere andere therapeutisch wirksame Stoffe.

Die Dosis kann innerhalb weiter Grenzen variieren und ist in jedem einzelnen Fall den individuellen Gegebenheiten anzupassen.

### Beispiele

### 1. Synthese der Oligonucleotide der Formel 1

### a) 3' aaaaaUGUCUACGUGUAUAGCUCCAC (Die mit kleinen Buchstaben gekennzeichneten Basen weisen eine 2'5'-Internucleosid-Bindung auf).

Die Synthesen wurden am ABI 394 DNA oder Expedite Synthesizer (Fa. Applied Biosystems, Weiterstadt) durchgeführt. Es wurden die vom Hersteller empfohlenen Synthesezyklen verwendet, wobei aber für die Ribonucleosid-2'-O-Phosphoramidite die Kondensation verdoppelt wurde (mit je 400 sec Kupplungszeit) und die Jodoxidation auf 30 sec verlängert wurde. Als Festphase wurde ein 1000 Å Controlled Pore Glass (CPG)-Träger eingesetzt, der 5'-O-Dimethoxytrityl-N-6-benzoyladenosin (NSS-6101-10A, Chemgenes, Waltham, MA) über die 2' bzw 3'-Position des Zuckers gebunden hielt. Nach Abspaltung der 5'-O-Dimethoxytrityl-Gruppe mit Trichloressigsäure wurde der 2'5'-verknüpfte Oligonucleotidteil durch viermalige Kondensation mit 5'-O-Dimethoxytrityl-N-6-benzoyl-3'-O-tert. Butyldimethylsilyl-adenosin-2'-O-Phosphoramidit (ANP-5681, Chemgenes, aufgebaut. Danach wurde der 3'5'-verknüpfte Oligonucleotidteil durch wiederholte Kondensation mit den entsprechenden 5'-O-Dimethoxytrityl-2'-O-tert. Butyldimethylsilyl-nucleosid-3'-O-Phosphoramiditen (ANP-5671 bis ANP-5680, Chemgenes) aufgebaut. Zur Abspaltung des Oligomers vom Träger und Entschützung der Phosphat- und Aminoschutzgruppen wurde der CPG-Träger mit 750 µL conc. Ammoniak/Ethanol (3:1, v:v) für 24 Stunden bei 30°C geschüttelt. Der Überstand wurde vom Träger getrennt und dieser nochmals zweimal mit 150 µL conc. Ammoniak/Ethanol (3:1, v:v) gewaschen. Die vereinigten Überstände wurden im Vakuum konzentriert und der Rückstand in 1200 µL Triethylaminx3HF (sehr giftig) zur Abspaltung der Silyl-Schutzgruppen für 24 Stunden bei 30°C geschüttelt. Dann gibt man 700 µL n-Butanol zu, kühlt das Gemisch für 30 Minuten auf Trockeneis und zentrifugiert. Das Pellet wurde noch zweimal mit Butanol gewaschen. Anschließend wurde noch eine Kochsalzfällung durchgeführt. Man erhielt 116 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8256.0, gef. 8256.8).

### b) 3' aaaaAUAUUACUUGCACUUAACGAG

Die Synthese wurde analog zu Beispiel 1a) durchgeführt, wobei der 2'5'-verknüpfte Oligonucleotidteil durch dreimalige Kondensation mit 5'-O-Dimethoxytrityl-N-6-benzoyl-3'-O-tert. Butyldimethylsilyl-adenosine-2'-O-Phosphoramidit (ANP-5681, Chemgenes) aufgebaut wurde. Man erhielt 112 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 7958.9, gef. 7958.6).

### c) 3' aaaaCCAUUUCAACAAGGUAAAAAAA

Die Synthese wurde analog zu Beispiel 1b) durchgeführt. Man erhielt 117 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8012.0, gef. 8011.8).

### d) 3' aaaaCUUCGCUUCCAACACCUAGAC

Die Synthese wurde analog zu Beispiel 1 b) durchgeführt. Man erhielt 117 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 7868.8, gef. 7868.6).

### e) 3' aaaaaaCUUCGCUUCCAACACCUAGAC

Die Synthese wurde analog zu Beispiel 1 a) durchgeführt, wobei der 2'5'-verknüpfte Oligonucleotidteil durch fünfmalige Kondensation mit 5'-O-Dimethoxytrityl-N-6-benzoyl-3'-O-tert. Butyldimethylsilyl-adenosin-2'-O-Phosphoramidit (ANP-5681, Chemgenes) aufgebaut wurde. Man erhielt 112 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8527.2, gef. 8527.5).

### f) 3' teg-AACUUCGCUUCCAACACCUAGAC (wobei teg einen Triethylenglycolphosphat-Rest bedeutet)

Die Synthese wurde analog zu Beispiel 1a) durchgeführt, wobei ein mit Triethylenglycolsuccinat derivatisierter CGP-Träger eingesetzt und der 2'5'-verknüpfte Oligonucleotidteil entsprechend der Sequenz durch Kondensation mit 5'-O-Dimethoxytrityl-N-6-benzoyl-3'-O-tert. Butyldimethylsilyl-adenosine-2'-O-Phosphoramidit (ANP-5681, Chemgenes) hergestellt wurde. Man erhielt 83 OD (260) des gewünschten Rohprodukts, das am 3'-terminalen Adenosin an der 2'-Position einen Triethylenglycolphosphatrest enthält. Das Produkt wurde weiter mittels HPLC und Elektrospray Massenspektrometrie (Negativmodus) charakterisiert (ber. 7422.5, gef. 7422.6).

### g) 5' GAAGCGAAGGUUGUGGAUCUGaaaa

Die Synthese wurde analog zu Beispiel 1b) durchgeführt. Man erhielt 108 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8149.0, gef. 8148.9).

### h) 5'-GGUAAAGUUGUUCCAUUUUUUaaaa

Die Synthese wurde analog zu Beispiel 1b) durchgeführt. Man erhielt 112 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 7930.7, gef. 7930.7).

### i) 5'-G A A G C G A A G G U U G U G G A U C U G

Die Synthese wurde analog zu Beispiel 1a) durchgeführt, wobei aber nur 3'5'-Internucleotid-Bindungen eingeführt wurden. Man erhielt 82 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 6832.2, gef. 6831.8).

### j) 3' a a a a-C*U*U*C G C*U*U C*C A A*C A C*C*U A G A*C

Die Synthese wurde analog zu Beispiel 1b) durchgeführt. Zur Einführung des Phosphorothioat-Restes wurde im jeweiligen Oxidationsschritt anstelle der Jodlösung das Beaucage-Reagenz (RN-1535, Chemgenes, Waltham, MA) eingesetzt. Man erhielt 112 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8029.4, gef. 8031.2).

### k) 3' a*a*a a-C*U*U*C G C*U*U C*C A A*C A C*C*U A G A*C

Die Synthese wurde analog zu Beispiel 1j) durchgeführt. Man erhielt 128 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8061.6, gef. 8062.8).

### I) 3' A*U*u a-C*U*U*C G C*U*U C*C A A*C A C*C*U A G A*C (enthält nur eine 2'5'-Internucleotidbindung zwischen u a)

Die Synthese wurde analog zu Beispiel 1k) durchgeführt. Man erhielt 96 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8015.5, gef. 8017.8).

### m) 3' a a a a-C U U C G C U U C C A A C A C C U A G A C (Die mit kleinen Buchstaben gekennzeichneten Basen weisen eine 2'5'-Internucleosid-Bindung auf; die unterstrichenen Nucleotide sind 2'-O-Methylribonucleotide)

Die Synthese wurde analog zu Beispiel 1a) durchgeführt. Im Falle der unterstrichenen Nucleotide wurden 5'-O-Dimethoxytrityl-2'-O-methyl-ribonucleosid-3'-O-Phosphoramidite (ANP-5751 bis ANP-5758, Chemgenes) kondensiert. Man erhielt 99 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8163.4.6, gef. 8165.1).

### n) 3' a*a*a a-C*U*U*C G C*U*U C*C A A*C A C*C*U A G A*C

Die Synthese wurde analog zu Beispiel 1m) durchgeführt. Man erhielt 127 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8356.1, gef. 8357.2).

### o) 3'-aaaaUAGUAGGACCUCUUGUAGAAA (edg-1-175_lo)

Die Synthese wurde analog zu Beispiel 1b) durchgeführt. Man erhielt 134 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8037.9, gef. 8038.9).

### p) 3'-aaaaGGUUCCGGUCGGCGUCGAGAC (edg-1-725_lo)

Die Synthese wurde analog zu Beispiel 1b) durchgeführt. Man erhielt 134 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8075.9, gef. 8076.9).

### q) 3' -aaaaGGUGCCUGUCUGCGGCGACAC (edg-1-mm_lo)

Die Synthese wurde analog zu Beispiel 1b) durchgeführt. Man erhielt 109 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 8035.9, gef. 8037.0).

### r) 5' AUCAUCCUGGAGAACAUCUUU (edg-1-175_up)

Die Synthese wurde analog zu Beispiel 1a) durchgeführt, wobei aber nur 3'5'-Internucleotid-Bindungen eingeführt wurden. Als Festphase wurde ein 1000 Å Controlled Pore Glass (CPG)-Träger eingesetzt, der 5'-O-Dimethoxytrityluridin (NSS-6104-10U, Chemgenes, Waltham, MA) über die 2' bzw 3'-Position des Zuckers gebunden hielt. Man erhielt 110 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 6618.0, gef. 6618.5).

### s) 5' AUCAUCCUGGAGAACAUCUUU-FITC (edg-1-175_up_FITC)

Die Synthese wurde analog zu Beispiel 1a) durchgeführt, wobei aber nur 3'5'-lntemucleotid-Bindungen eingeführt wurden. Als Festphase wurde ein 500 Å Controlled Pore Glass (CPG)-Träger eingesetzt, der ein geschütztes Fluorescein-Derivat enthält (NSS-97505-A1 CL, Chemgenes, Waltham, MA). Man erhielt 79 OD (260) des gewünschten Rohprodukts, das bei der Gelelektrophorese nur eine Hauptbande zeigt. Das Produkt wurde weiter mittels HPLC und Elektrospray Massenspektrometrie (Negativmodus) charakterisiert (ber. 7428.7, gef. 7432.3).

### 2. Inhibition der Luciferase-Expression in SL-3 Zellen

Zur Prüfung auf biologische Aktivität wurden folgende Oligonucleotide hergestellt, gegebenenfalls zum Doppelstrang hybridisiert, und auf Inhibition der Luciferase-Aktivität in folgenden Test-Ansätzen geprüft.

| | | | Test-Ansatz |
|---|---|---|---|
| 3' | aaaaCCAUUUCAACAAGGUAAAAAA | luc-1087_lo | 1 |
| 5' | GGUAAAGUUGUUCCAUUUUUUaaaa | luc-1087_up | 2 |
| 5' | GGUAAAGUUGUUCCAUUUUUUaaaa | luc-1087_up | 3 |
| 3' | aaaaCCAUUUCAACAAGGUAAAAAA | luc-1087_lo | |
| 5' | GGUAAAGUUGUUCCAUUUUUU | luc-1087_up | 4 |
| 3' | aaaaCCAUUUCAACAAGGUAAAAAA | luc-1087_lo | |
| 3' | aaaaCUUCGCUUCCAACACCUAGAC | luc-1108_lo | 5 |
| 5' | GAAGCGAAGGWGUGGAUCUGaaaa | luc-1108_up | 6 |
| 5' | GAAGCGAAGGWGUGGAUCUGaaaa | luc-1108_up | 7 |
| 3' | aaaaCUUCGCUUCCAACACCUAGAC | luc-1108_lo | |
| 5' | GAAGCGAAGGUUGUGGAUCUG | luc-1108_up | 8 |
| 3' | aaaaCUUCGCUUCCAACACCUAGAC | luc-1108_lo | |
| 5' | GAAGCGAAGGWGUGGAUCUGaaaa | luc-1108_up | 9 |
| 3' | aaaaaaCWCGCUUCCAACACCUAGAC | luc-1108_lo | |
| 5' | GAAGCGAAGGWGUGGAUCUG | luc-1108_up | 10 |
| 3' | aaaaaaCUUCGCUUCCAACACCUAGAC | luc-1108_lo | |
| 5' | GAAGCGAAGGWGUGGAUCUGaaaa | luc-1108_up | 11 |
| 3' | teg-aaCWCGCWCCAACACCUAGAC | luc-1108_lo-teg | |
| 5' | GGUAAAGUUGUUCCAUUUUUUaaaa | luc-1087_up | 12 |
| 3' | aaaaCWCGCWCCAACACCUAGAC | luc-1108_lo | |
| 3' | aaaaCCAUUUCAACAAGGUAAAAAA | luc-1087_lo | 13 |
| 3' | aaaaCWCGCWCCAACACCUAGAC | luc-1108_lo | |

Die Testansätze 1, 2, 5, 6, 12 und 13 enthalten RNA, die nicht als Doppelstrang vorliegt. Dagegen liegen in den Testansätzen 3, 4, 7, 8, 9, 10, und 11 die Oligonucleotide gepaart als Doppelstrang vor. Die Doppelstränge 7, 9 und 11 weisen an beiden Strängen einen überhängenden 2'5'-verknüpften Oligonucleotid-Rest auf. Das Oligoribonucleotid luc-1108_lo enthält am 3'-Ende nur zwei überhängende 2'5'-verknüpfte Nucleotide sowie einen Triethylenglycolphosphat-Rest (teg). Die Doppelstränge der Testansätze 8 und 10 weisen nur an einem Strang einen 2'5'-verknüpften Überhang auf.

Transfektion: Am Vortag des Experiments wurden 2x10⁶ Zellen/mL in 6-Well Platten ausplattiert. Die Oligonucleotide wurden durch Erhitzen der beiden Stränge in verdünntem Puffer und Abkühlen zum Doppelstrang hybridisiert und in 100µL SF 90011 SFM (SF-900 Serumfreies Insektenmedium II; Gibco BRL 10902-096) aufgenommen. Zur Transfektion wurden 10µL Lipofectin (1mg/mL; Gibco BRL) mit 100µL SF 900II SFM gemischt und 15 min bei Raumtemperatur inkubiert. Danach wurden der Lipofectin-Mix und die Nucleinsäure zusammen pipettiert und 15-45 min bei Raumtemperatur inkubiert. In der Zwischenzeit wurden die Zellen mit 3ml serumfreien Medium gewaschen, und nacheinander mit 800µL SF 90011 SFM und der Nucleinsäure/Lipofectin-Mischung versetzt und über Nacht bei 25 Grade inkubiert. Am nächsten Tag gibt man 1 mL Medium und + Serum (Gibco BRL 10122-166; Endkonzentration 2%) zu.

Dual-Luciferase Reporter (DLR; Promega E1960) Assay System: (http://www.promega.com/catalog/CatalogProducts.asp?catalog%5Fname=Promega %5FProducts&category%5Fname=Dual%2DLuciferase+Reporter+Assay+System&de scription%5Ftext=Dual%2DLuciferase%3Csup%3E%26reg%3B%3C%2Fsup%3E+R eporter+Assay+System)

Der DLR Assay von Promega erlaubt die sequenzielle Bestimmung der Aktivität der Firefly Luciferase und Renilla Luciferase, die sich in ihrer Nucleinsäuresequenz unterscheiden, aus einer einzigen Probe. Die zu messenden Oligonucleotide gemäß Formel I waren gegen die Firefly Luciferase gerichtet. Es sollte also nur die Firefly Luciferase, aber nicht die Renilla Luciferase Aktivität gehemmt werden. Neben der Hemmwirkung kann also auch auf Spezifität geprüft werden.

Zur passiven Lyse der Zellen in den Well- Plates wurde zunächst das Medium entfernt und die Zellen mit PBS (Phosphatgepufferte Kochsalzlösung (Gibco BRL 14200-067) gewaschen. Das Medium wurde komplett abgesaugt und anschließend der PLB (Passiver Lysis Puffer, 1:5 mit Wasser verdünnt; 500µL PLB (1x) in ein Well einer 6-Well Platte geben) dazu gegeben. Danach wurde 15 Minuten unter Schütteln bei Raumtemperatur inkubiert.

Zur Präparation des Luciferase Assay Reagenz II ( LARII) wurde das Luciferase Assay Substrat (LAS) in 10 mL des Luciferase Assay Puffers II (LAB II) resuspendiert. Zur Präparation des Stop & Glo Reagenz wurden 200µL des Stop & Glo Substrat (Lösung) in das Fläschen, welches trockenes Stop & Glo Substrat enthielt, gegeben und 10 Sekunden mit dem Vortexer durchmischt. Um eine 1 x Stop& Glo Lösung zu bekommen, addiert man 20µL des 50x Stop & Glo Substrat und 1 mL Stop & Glo Puffer. Dies ist ausreichend für 10 Assays.

DLR-Assay: 100µL LAR II wurden zusammen mit 20µL Zell Lysat in ein Well gegeben und durch Auf- und Abpipettieren für 2-3 Sekunden gemischt. Nach der luminometrischen Messung der Firefly-Luciferaseaktivität wurden 100µL Stop & Glo Reagenz zugegeben, gemischt und dann die Renilla-Luciferase-Aktivität gemessen. Zur Bestimmung der Lumineszenz wurde das Luminometer Fluoroskan Ascent FL (Fa. Thermo Labsystems, Frankfurt) verwendet.

| Testansatz | Typ | % Inhibition der Firefly-Luciferase |
|---|---|---|
| 1 | ss | 13 |
| 2 | ss | 12 |
| 3 | ds | 44 |
| 4 | ds | 53 |
| 5 | ss | 19 |
| 6 | ss | 17 |
| 7 | ds | 56 |
| 8 | ds | 57 |
| 9 | ds | 42 |
| 10 | ds | 51 |
| 11 | ds | 20 |
| 12 | ss | 7 |
| 13 | ss | 18 |

| | | |
|---|---|---|
| ss bedeutet Einzelstrang, ds bedeutet Doppelstrang | | |

Die doppelsträngigen Oligonucleortide (3,4, 7-10) hemmten die Firefly-Luciferase-Aktivität wesentlich stärker als die entsprechenden einzelsträngigen Moleküle (1,2, 5,6, 12 und 13). Ausnahme ist der Doppelstrang mit nur 2 überhängenden Nucleotiden im unteren Strang (Testansatz 11). Ein überhängender 2'5'-(A)₄ Rest im oberen Strang hatte keinen oder nur sehr geringen positiven Einfluss auf die Aktivität des Doppelstrangs, sofern sich im unteren Strang ein 2'5'-(A)₄ Rest befand (vgl. 3 vs. 4 und 7 vs. 8). Ein 2'5'-(A)₄ Rest im unteren Strang führte zu einer signifikant besseren Wirkung des Doppelstrangs als ein 2'5'-(A)₂ Rest (vgl 7 bzw. 8 vs. 11). 5). Zwei einzelsträngige 2'5'-(A)₄ -Oligonucleotide im Gemisch, die aufgrund der Nichtkomplementarität der Basen keinen Doppelstrang ausbilden konnten (12 und 13), waren viel weniger wirksam als Doppelstränge mit komplementären Basen und überhängenden 2'5'-Adenylat-Resten.

Es wurden auch folgende modifizierte Oligonucleotide der Formel I in den Testansätzen 14 bis 18 getestet.

* bedeutet Phosphorothioat, N: 2'-O-methyl-RNA, aaaa: 2'5'-verknüpftes Adenylat

| Testansatz | Typ | % Inhibition der Firefly-Luciferase |
|---|---|---|
| 14 | ds | 67 |
| 15 | ds | 2 |
| 16 | ds | 93 |
| 17 | ds | 0 |
| 18 | ss | 0 |
| 1100 bp dsRNA | | 94 |
| Ohne dsRNA | | 0 |

| | | |
|---|---|---|
| ss bedeutet Einzelstrang, ds bedeutet Doppelstrang | | |

Die Einführung von Phosphorothioat-Resten an bestimmten Positionen im unteren (Testansatz 14) oder im unteren und oberen Strang (Testansatz 16) führte zu einer signifikant besseren Wirkung der erfindungsgemässen Oligonucleotide, während die Einführung von 2'-O-Methyl-Resten im gesamten unteren oder oberen Strang (Testansätze 15 und 17) zu einer starken Absenking der Aktivität führte.

Überraschenderweise hemmte das kleine Oligonucleotid des Testansatzes 16 die Expression der Firefly Luciferase ebenso gut wie die sehr lange (ca. 1100 bp) doppelsträngige RNA. Der Einzelstrang (Testansatz 18) war ebenfalls unwirksam.

### 3. Inhibition der edg-1 Expression in primären menschlichen Nabelschnur-Zellen (HUVEC).

Um zu zeigen, dass die erfindungsgemässen Oligonucleotide auch in primären menschlichen Zellen zur Inhibition der Genexpression eingesetzt werden können, wurden diese auch gegen ein menschliches Gen bzw. die zugehörige RNA gerichtet und an menschlichen Zellen (HUVEC, human umbilical vein endothelial cells) getestet.
Die entsprechenden Oligonucleotide wurden synthetisiert.

| | | Test-Ansatz |
|---|---|---|
| 5' AUCAUCCUGGAGAACAUCUUU | edg-1-175_up | 14 |
| 3'-aaaaUAGUAGGACCUCUUGUAGANA | edg-1-175_lo | |
| 5' CCAAGGCCAGCCGCAGCUCUG | edg-1-725_up | 15 |
| 3'-aaaaGGUUCCGGUCGGCGUCGAGAC | edg-1-725_lo | |
| | | |
| Mismatch Kontrolle | | |
| 5' CCACGGACAGACGCCGCUGUG | edg-1-mm_up | 16 |
| 3'-aaaaGGUGCCUGUCUGCGGCGACAC | edg-1-mm_up | |
| 5' AUCAUCCUGGAGAACAUCUUU-FITC | edg-1-175_up_FITC | 17 |
| 3'-aaaaUAGUAGGACCUCUUGUAGAAA | edg-1-175_lo | |

Die doppelsträngigen Oligonucleotide der Testansätze 14 bis 16 enthalten nur in einem Strang (nichtcodierend) einen 2'5'-verknüpften überhängenden Rest. Dieses Merkmal ist auch eine besondere Ausgestaltung dieser Erfindung.

Zellen (HUVECs) und Detektion der zellulären Aufnahme. Transfektion: 24h vor der eigentlichen Transfektion wurden primäre HUVECs (2. Passage, isoliert nach Jaffe et al., 1973, J. Clin.Invest 52, pp.2745) in einer Dichte von 2,5 x 10⁵ Zellen/Well in Ratten Kollagen-I (Biocoat, #354400, Becton Dickinson) beschichtete 6-Well Platten ausplattiert. Strang und Gegenstrang der jeweiligen Oligonucleotide (jeweils 1 mM in sterilfiltriertem PBS, pH 7.4, Gibco BRL # 14200-067) wurden äquimolar gemischt und durch fünfminütige Inkubation bei 95 °C, einer anschließenden Abkühlung auf Raumtemperatur und fünfminütigen Inkubation bei 4° C hybridisiert. Zur Transfektion wurden 6 µl Lipofectin (1 mg/ml; Gibco BRL, # 18292-011) mit 200 µl serumfreien Opti-MEM 1-Medium ( Gibco BRL, ,31985-047) gemischt und 15 Minuten bei Raumtemp-eratur inkubiert. Parallel hierzu wurden eine 10 µM (→ Endkonzen-tration 0,1 µM) bzw. eine 100 µM (→ Endkonzentration 1 µM) hybridisierte Oligonucleotid-lösung (in PBS, pH 7,4) im Verhältnis 1:10 mit serumfreien Opti-MEM 1-Medium verdünnt und im selben Volumen mit der präinkubierten Lipofectinlösung gemischt. Nach 15 minütiger Inkubation bei Raumtemperatur wurde dieser Ansatz auf 2 ml mit serumfreien Opti-MEM 1-Medium aufgefüllt und der einmal mit PBS gewaschene Zellrasen für 4 Stunden bei 37°C, 5% CO₂ und 95% Luftfeuchtigkeit mit diesem Ansatz inkubiert. Anschließend wurde der erneut mit PBS gewaschene Zellrasen mit Serum-haltigen EGM-Medium (CellSystems, # CC-3024 + EGM Zusätze # CC-3124) überschichtet und für weitere 24 bzw. 48 h inkubiert. Im Falle der Aufnahmestudien mit fluoreszenzmarkierten Oligonucleotiden wurden die Zellen nach 4 stündiger Inkubation mit 5% Paraformaldehyd (in PBS, pH 7.4) fixiert und direkt mit einem inversen Fluoreszenzmikroskop (Zeiss Axiovert 135M) durch Anregung mit einem FITC-Filter (Excitation: 490 nm, Emission: 510 nm) in einer 200-fachen Vergrößerung mit einer gekühlten CCD-Camera (ORCA-1, Bfi optilas) aufgenommen und durch die AQM2000-Software (Kinetic Imaging) prozessiert.
Western Blot Analyse: Zur Lyse der Zellen wurde der Zellrasen einmal mit PBS gewaschen und anschließend mit 200 µl/well 2 x Laemmli-Puffer (Bio-Rad #161-0737) überschichtet. Nach fünfminütiger Inkubation bei Raumtemperatur wurde das Zelllysat mit einem Zellschaber (Becton Dickinson, #3085) ge-sammelt und vor der diskontinuierlichen 12%-SDS-Polyacrylamidgelelektro-phorese (SDS-PAGE, Laemmli et al., 1970, Bio-Rad-Criterion-System #345-0014) für 5 Minuten bei 95°C gekocht und hiervon 45 µl pro Tasche aufgetragen. Der Gellauf erfolgte in 1 x Tris/Glycine/SDS Puffer (Bio-Rad # 161-0732). Für den Immunoblot wurde das Gel mit Hilfe der Bio-Rad Criterion Westernblot Apparatur (#170-4070) auf eine Nitrocellulose- (NC) Membran (Amersham # RPN 2020D) in 1 x Tris/Glycine Puffer (Bio-Rad #161-0732, + 10% Methanol) transferiert. Anschließend wurde die NC-Membran für eine Stunde bei Raumtemperatur mit 1 x TBS-Puffer (Bio-Rad # 170-6435), der 5% Milchpulver ("Blotto", Bio-Rad #170-6404) und 0,1% Tween 20 (Bio-Rad # 170-6531) enthielt abgesättigt. Nach dreimaligem Waschen der Membran in Blotto-freiem TBS-Tween (TBST) Puffer wurde die Membran mit dem anti-hEDG-1 Primärantikörper (polyklonales Kaninchenserum, das durch Immunisierung mit der EDG-1-spezifischen Peptidsequenz CKAHRSSVSDYVNYD, gekoppelt an KLH, gewonnen und gegen die o.g. Peptidsequenz affinitätsgereinigt wurde) über Nacht bei 4°C in einer 1:50 Verdünnung in TBST-Blotto inkubiert. Nach dreimaligem Waschen mit TBST wurde der Sekundärantikörper (anti-Kaninchen, alkalische Phosphatase gekoppellt, Dianova # 111-055-045) in einer 1:2000 Verdünnung in TBST-Blotto für eine Stunde bei Raumtemperatur inkubiert. Nach erneutem Waschen (Siehe oben) erfolgte die ECF ("enhanced chemifluorescent")-Detektionsreaktion (Amersham #RPN5785), wobei die mit Plastikfolie abgedeckte NC-Membran mit 1 ml ECF-Substrat (Amersham Pharmacia #RPN5785) für 5 Minuten bei Raumtemperatur inkubiert und mit einem Fluor-Imager 595 Scanner (Amersham Pharmacia) detektiert wurde. Die Quantifizierung erfolgte mit der ImageQuant Software (Amersham Pharmacia), wobei gegen das β-Tubulinsignal normalisiert wurde, das nach einmaligem Entfärben (Alpha Diagnostic Kit # 90100) der NC-Membran und durch Inkubation mit dem β-Tubulin spezifischen Primärantikörper (affinitäts-gereinigter Kaninchen Antikörper, Santa Cruz # sc-9104) nach der oben beschriebenen Methode, erhalten wurde.

| | | EDG-1 Protein ( % der Kontrolle) | |
|---|---|---|---|
| Konzentration der ds RNA (µM) | Test-Ansatz 14 (Region "175") | Test-Ansatz 15 (Region "725") | Test-Ansatz 16 (mismatch) |
| 0.0 | 100 | 100 | 100 |
| 0.1 | 51 | 121 | 119 |
| 1.0 | 23 | 51 | 118 |

Die Behandlung der primären HUVEC-Zellen mit den erfindungsgemässen doppelsträngigen Oligonucleotiden führte zu einer dosisabhängiggen Hemmung der Expression von edg-1. Die Hemmung erwies sich als Zielgen-spezifisch, da nur die Edg-1 Protein-Spiegel, aber nicht die Tubulin-Spiegel nach Behandlung mit den edg-1-spezifischen Oligonucleotiden in den Testansätzen 14 und 15 abgesenkt waren. Die Hemmung erwies sich auch als sequenzspezifisch hinsichtlich der eingesetzten Oligonucleotide, da nur die zu edg-1 homologen Oligonucleotide der Test-Ansätze 14 und 15 die Expression von edg-1 hemmten, während die doppelsträngige Nucleinsäure des Test-Ansatzes 16, die sich in 5 Nucleotiden von der edg-1 Sequenz unterscheidet, die edg-1 Expression nicht hemmte.

Unseres Wissens ist dies das erste Experiment, das die sequenzspezifische Hemmung der Genexpression mit doppelsträngiger RNA in primären menschlichen Zellen beschreibt.

Mit Hilfe des fluoreszenzmarkierten doppelsträngigen Oligonucleotids des Test-Ansatzes 17 wurde die zelluläre Aufnahme in HUVEC Zellen überprüft. Nach 4 Stunden Inkubation von Test-Ansatz 17 wurde mit Hilfe der Fluoreszenzmikrospkopie eine gute zelluläre Aufnahme festgestellt. Das aufgenommene doppelsträngige Oligonucleotid befand sich hauptsächlich im Zytoplasma, während ein einzelsträngiges FITC-markiertes Oligonucleotid unter gleichen Bedingungen hauptsächlich im Zellkern zu finden war.

### 4. Inhibition der edg-1 Expression in primären menschlichen Nabelschnur-Zellen (HUVEC) mit Hilfe von Phosphorothioat-modifizierten Oligomeren.

Die mit Phosphorothioat an bestimmten Positionen modifizierten Oligoribonucleotid-Analoga wurden wie in Beispiel 3 beschrieben in primären menschlichen Zellen zur Inhibition der Genexpression von Edg-1 in menschlichen Zellen (HUVEC, human umbilical vein endothelial cells) eingesetzt. wobei * Phosphorothioat; (a*a*a a) ein 2'5'-verknüpftes Adenylat (partiell mit * modifiziert), und teg ein Triethyleneglycolphosphat ist.

Die doppelsträngigen Oligoribonucleotide der Testansätze 18 bis 20 enthalten nur in einem Strang (nichtcodierend) einen 2'5'-verknüpften überhängenden Rest, wobei nur bestimmte Internucleotidbindungen mit Phosphorothioat modifiziert sind. Dieses Merkmal ist ebenfalls eine besondere Ausgestaltung dieser Erfindung.

| | | EDG-1 Protein ( % der Kontrolle) | |
|---|---|---|---|
| Konzentration der ds RNA (µM) | Test-Ansatz 18 (Region "175") | Test-Ansatz 19 (Region "725") | Test-Ansatz 20 (mismatch) |
| 0.0 | 100 | 10 | 100 |
| 0.1 | 46 | 37 | 112 |
| 1.0 | 27 | 27 | 109 |

Dieser Versuch wurde mit einer größeren Variation der Dosierung wiederholt.

| | | EDG-1 Protein ( % der Kontrolle) | |
|---|---|---|---|
| Konzentration der ds RNA (µM) | Test-Ansatz 18 (Region "175") | Test-Ansatz 19 (Region "725") | Test-Ansatz 20 (mismatch) |
| 0 | 100,0 | 100,0 | 100,0 |
| 0,01 | 107,7 | 95,6 | 93,4 |
| 0,05 | 71,9 | 93,4 | 115,7 |
| 0,1 | 49,9 | 61,7 | 100,8 |
| 0,5 | 43,5 | 25,8 | 125,0 |
| 1,0 | 25,1 | 12,8 | 113,5 |

Die Behandlung der primären HUVEC-Zellen mit den erfindungsgemässen chemisch modifizierten doppelsträngigen Oligoribonucleotiden führte zu einer dosisabhängiggen Hemmung der Expression von edg-1. Die Hemmung erwies sich als Zielgen-spezifisch, da nur die Edg-1 Protein-Spiegel, aber nicht die Tubulin-Spiegel nach Behandlung mit den edg-1-spezifischen Oligonucleotiden in den Testansätzen 18 und 19 abgesenkt waren. Die Hemmung erwies sich auch als sequenzspezifisch hinsichtlich der eingesetzten Oligonucleotide, da nur die zu edg-1 homologen Oligonucleotide der Test-Ansätze 18 und 19 die Expression von edg-1 hemmten, während die doppelsträngige Nucleinsäure des Test-Ansatzes 20, die sich in 5 Nucleotiden von der edg-1 Sequenz unterscheidet, die edg-1 Expression nicht hemmte.

## Patentansprüche

1. Oligonucleotid der Formel I 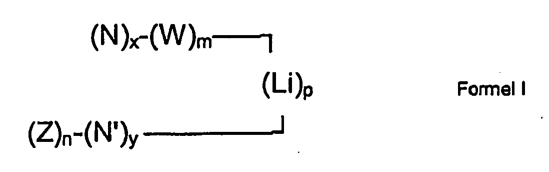 wobei
N und N' natürlich oder nicht natürlich vorkommende Nucleotide sind, von denen 21 Nukleotide zueinander komplementär sind und wobei der Nucleotidstrang (N')_{y} der nicht-kodierende Strang, bzw. der zum Ziel-Gen komplementäre Strang ist,
x und y gleich je 21 sind,
n unabhängig gleich 4 bis 6 ist,
m unabhängig gleich 0 bis 20 ist,
p unabhängig gleich 0 bis 10 ist,
W und Z natürlich oder nicht natürlich vorkommende Nucleotide sind, die über eine 3'5'- oder 2'5'-Internucleosid-Bindung verknüpft sind,
Li ein Linker ist, der die beiden Nucleotidstränge kovalent verknüpft,
**dadurch gekennzeichnet, dass** mindestens vier Reste Z und gegebenenfalls zwei Reste W über eine 2'5'-Internucleosid-Bindung verknüpft sind und einzelsträngig vorliegen.

2. Oligonukleotid der Formel I nach Anspruch 1, wobei m 0 bis 10 ist.

3. Oligonukleotid der Formel I nach Anspruch 2, wobei m 0 bis 6 ist.

4. Oligonukleotid der Formel I nach Anspruch 3, wobei m gleich 0 ist.

5. Oligonukleotid der Formel I nach einem oder mehreren der Ansprüche 1 bis 4, wobei p 0 bis 5 ist.

6. Oligonucleotide der Formel I nach Anspruch 5, wobei p gleich null ist.

7. Oligonucleotid der Formel I nach einem oder mehreren der Ansprüche 1 bis 6, mit der Maßgabe, dass deren homologe Ziel-RNA eines der folgenden Sequenzmuster aufweist.
5'-(U)ᵥ-(M)_{z}-(U)_{w}
5'-(U)ᵥ-(M)_{z}-UX
5'-UX-(M)_{z}-UX und
5'-(U)ᵥ-(M)_{z}.
wobei v unabhängig gleich 2 bis 20 ist,
wobei w unabhängig gleich 2 bis 20 ist,
z unabhängig 15 bis 25 ist,
U ein Uridin ist und M gleich A, G, C oder U und X gleich A, G oder C sind.

8. Oligonucleotid der Formel I nach Anspruch 7, wobei v 2 bis 10 ist.

9. Oligonucleotid der Formel I nach Anspruch 7 oder 8, wobei v 2 bis 6 ist.

10. Oligonucleotid der Formel I nach einem oder mehreren der Ansprüche 7 bis 9, wobei w 2 bis 10 ist.

11. Oligonucleotid der Formel I nach Anspruch 10, wobei w 2 bis 6 ist.

12. Oligonucleotid der Formel I nach einem oder mehreren der Ansprüche 7 bis 11, wobei z 16 bis 23 ist.

13. Oligonucleotid der Formel I nach Anspruch 12, wobei z 19 bis 21 ist.

14. Oligonucleotid der Formel I nach einem oder mehreren der Ansprüche 1 bis 13, wobei Z für Adenosin oder 3'-Deoxyadenosin steht.

15. Oligonucleotid der Formel I nach einem oder mehreren der Ansprüche 1 bis 14, in denen eine oder mehrere natürliche Phosphodiester-Brücken durch unnatürliche Internucleotid-Brücken, welche gegen Nuclease-Abbau stabilisieren, ersetzt sind.

16. Oligonucleotid der Formel I nach einem oder mehreren der Ansprüche 1 bis 15, in denen eine oder mehrere natürliche Phosphodiester-Bindungen durch Phosphorothioat-Bindungen ersetzt sind.

17. Oligonucleotid der Formel I nach einem oder mehreren der Ansprüche 1 bis 16, in denen mehrere natürliche Phosphodiester-Bindungen durch Phosphorothioat-Bindungen ersetzt sind, wobei sich diese Modifikationen an den Enden und internen Pyrimidin-Nucleotiden befinden.

18. Oligonucleotid der Formel I nach einem oder mehreren der Ansprüche 1 bis 5 oder 7 bis 17, wobei Li ein natürlich oder nicht natürlich vorkommendes Nucleotid ist.

19. Oligonucleotid der Formel I nach einem oder mehreren der Ansprüche 1 bis 5 oder 7 bis 17, wobei Li ein nicht nucleotidischer Linker ist.

20. Verfahren zur Hemmung der Genexpression eines Ziel-Gens in einer Zelle in vitro unter Zuhilfenahme eines oder mehrer Oligonucleotide nach einem oder mehreren der Ansprüche 1 bis 19, wobei zuerst ein zu einem entsprechenden Ziel-Gen komplementäres Oligonukleotid hergestellt wird, dieses Oligonukleotid in eine Zelle eingeführt wird, die Zelle inkubiert wird und anschließend die Hemmung der Genexpression des Ziel-Gens durch Vergleichsmessungen an der Menge der entsprechenden mRNA oder des entsprechenden Genprodukts in einer Kontrollzelle bestimmt wird.

21. Verfahren nach Anspruch 20 zur Hemmung der Genexpression eines Ziel-Gens in einer Zelle, in der die 2'5'-Oligoadenylat-Synthase im Vergleich zu einer Kontrollzelle unterexprimiert oder defekt ist.

22. Arzneimittel, enthaltend ein Oligonucleotid nach einem oder mehreren der Ansprüche 1 bis 19 sowie Zusatz- und/oder Trägerstoffe und gegebenenfalls Hilfsstoffe zur Herstellung oder Formulierung eines Arzneimittels.

23. Verwendung eines Oligonucleotids nach einem oder mehreren der Ansprüche 1 bis 19 zur Identifizierung oder Validierung neuer therapeutischer Ziel-Gene.

24. Verwendung eines Oligonucleotids nach einem oder mehreren der Ansprüche 1 bis 19 zur Identifizierung oder Validierung neuer Ziel-Gene in der Pflanzenschutz-Forschung.

25. Verfahren zur Herstellung eines Oligonucleotids nach einem oder mehreren der Ansprüche 1 bis 19, wobei die Oligonucleotide zuerst in Lösung oder an der Festphase durch sukzessive oder blockweise Kupplung hergestellt, gegebenenfalls zum Doppelstrang hybridisiert werden und nach der Herstellung isoliert und gereinigt werden.

26. Verfahren zur Herstellung eines Arzneimittels, wobei ein Oligonucleotid-Derivat gemäß Anspruch 25 hergestellt wird und gegebenenfalls mit weiteren Zusatz- und/oder Trägerstoffen und gegebenenfalls Hilfsstoffen versetzt wird.

## Claims

1. An oligonucleotide of the formula I 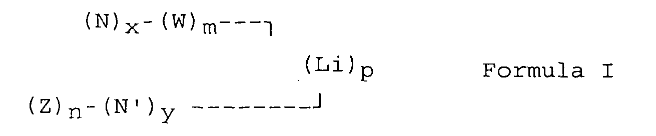 where
N and N' are naturally or not naturally occurring nucleotides of which 21 nucleotides are complementary to one another and where the nucleotide strand (N')_{y} is the non-coding strand, or the strand complementary to the target gene,
x and y are each 21,
n is independently 4 to 6,
m is independently 0 to 20,
p is independently 0 to 10,
W and Z are naturally or not naturally occurring nucleotides which are linked via a 3'5' or 2'5' internucleoside bond,
Li is a linker which covalently links the two nucleotide strands,
wherein at least four residues Z and, where appropriate, two residues W are linked via a 2'5' internucleoside bond and are present in single-stranded form.

2. The oligonucleotide of the formula I as claimed in claim 1, wherein m is 0 to 10.

3. The oligonucleotide of the formula I as claimed in claim 2, wherein m is 0 to 6.

4. The oligonucleotide of the formula I as claimed in claim 3, wherein m is 0.

5. The oligonucleotide of the formula I as claimed in one or more of claims 1 to 4, wherein p is 0 to 5.

6. The oligonucleotide of the formula I as claimed in claim 5, wherein p is zero.

7. The oligonucleotide of the formula I as claimed in one or more of claims 1 to 6, with the proviso that its homologous target RNA has one of the following sequence patterns.
5'-(U)ᵥ-(M)_{z}-(U)_{w}
5'-(U)ᵥ-(M)_{z}-UX
5'-UX-(M)_{z}-UX and
5'-(U)ᵥ-(M)_{z},
where v is independently 2 to 20,
where w is independently 2 to 20,
z is independently 15 to 25,
U is a uridine and M is A, G, C or U and X is A, G or C.

8. The oligonucleotide of the formula I as claimed in claim 7, wherein v is 2 to 10.

9. The oligonucleotide of the formula I as claimed in either of claims 7 and 8, wherein v is 2 to 6.

10. The oligonucleotide of the formula I as claimed in one or more of claims 7 to 9, wherein w is 2 to 10.

11. The oligonucleotide of the formula I as claimed in claim 10, wherein w is 2 to 6.

12. The oligonucleotide of the formula I as claimed in one or more of claims 7 to 11, wherein z is 16 to 23.

13. The oligonucleotide of the formula I as claimed in claim 12, wherein z is 19 to 21.

14. The oligonucleotide of the formula I as claimed in one or more of claims 1 to 13, wherein Z is adenosine or 3'-deoxyadenosine.

15. The oligonucleotide of the formula I as claimed in one or more of claims 1 to 14, in which one or more natural phosphodiester bonds have been replaced by unnatural internucleotide bonds which stabilize against nuclease degradation.

16. The oligonucleotide of the formula I as claimed in one or more of claims 1 to 15, in which one or more natural phosphodiester bonds have been replaced by phosphorothioate bonds.

17. The oligonucleotide of the formula I as claimed in one or more of claims 1 to 16, in which a plurality of natural phosphodiester bonds have been replaced by phosphorothioate bonds, with said modifications being located on the ends and on internal pyrimidine nucleotides.

18. The oligonucleotide of the formula I as claimed in one or more of claims 1 to 5 or 7 to 17, wherein Li is a naturally or not naturally occurring nucleotide.

19. The oligonucleotide of the formula I as claimed in one or more of claims 1 to 5 or 7 to 17, wherein Li is a non-nucleotide linker.

20. A method for inhibiting gene expression of a target gene in a cell in vitro with the aid of one or more oligonucleotides as claimed in one or more of claims 1 to 19, wherein first an oligonucleotide complementary to an appropriate target gene is prepared, said oligonucleotide is introduced into a cell, said cell is incubated and inhibition of the gene expression of the target gene is then determined by comparative measurements of the amount of the corresponding mRNA or corresponding gene product in a control cell.

21. The method as claimed in claim 20 for inhibiting gene expression of a target gene in a cell in which 2'5'-oligoadenylate synthase is underexpressed in comparison with a control cell or is defective.

22. A pharmaceutical comprising an oligonucleotide as claimed in one or more of claims 1 to 19 and also additives and/or carriers and, where appropriate, excipients for preparing or formulating a pharmaceutical.

23. The use of an oligonucleotide as claimed in one or more of claims 1 to 19 for identifying or validating novel therapeutic target genes.

24. The use of an oligonucleotide as claimed in one or more of claims 1 to 19 for identifying or validating novel target genes in crop protection research.

25. A method for preparing an oligonucleotide as claimed in one or more of claims 1 to 19, wherein the oligonucleotides are first prepared in solution or on the solid phase by successive coupling or coupling in blocks, are, where appropriate, hybridized to give a double strand and are, after the preparation, isolated and purified.

26. A method for preparing a pharmaceutical, wherein an oligonucleotide derivative as claimed in claim 25 is prepared and, where appropriate, admixed with further additives and/or carriers and, where appropriate, excipients.

## Revendications

1. Oligonucléotide de formule I 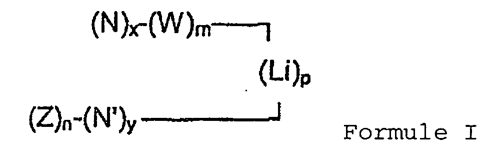 où
N et N' sont des nucléotides naturels ou non naturels, dont 21 nucléotides sont complémentaires les uns aux autres et où le brin de nucléotides (N')_{y} est le brin non codant ou le brin complémentaire au gène cible,
x et y valent chacun 21,
n vaut, indépendamment, 4 à 6,
m vaut, indépendamment, 0 à 20,
p vaut, indépendamment, 0 à 10,
W et Z sont des nucléotides naturels ou non naturels, qui sont liés via une liaison internucléoside 3'5' ou 2'5',
Li est un lieur qui lie les deux brins de nucléotides par covalence,
**caractérisé en ce qu'**au moins quatre résidus Z et le cas échéant deux résidus W sont liés via une liaison internucléoside 2'5' et se trouvent sous forme de monobrin.

2. Oligonucléotide de formule I selon la revendication 1, où m vaut 0 à 10.

3. Oligonucléotide de formule I selon la revendication 2, où m vaut 0 à 6.

4. Oligonucléotide de formule I selon la revendication 3, où m vaut 0.

5. Oligonucléotide de formule I selon l'une quelconque des revendications 1 à 4, où p vaut 0 à 5.

6. Oligonucléotide de formule I selon la revendication 5, où p vaut zéro.

7. Oligonucléotide de formule I selon l'une ou plusieurs des revendications 1 à 6, à condition que leur ARN cible homologue présente un des motifs de séquence suivants :
5'-(U)ᵥ-(M)_{z}-(U)_{w}
5'-(U)ᵥ-(M)_{z}-UX
5'UX-(M)_{z}-UX et
5'-(U)ᵥ-(M)_{z}
où v vaut, indépendamment, 2 à 20,
où w vaut, indépendamment, 2 à 20,
z vaut, indépendamment, 15 à 25,
U représente une uridine et M représente A, G, C ou U et X représente A, G ou C.

8. Oligonucléotide de formule I selon la revendication 7, où v vaut 2 à 10.

9. Oligonucléotide de formule I selon la revendication 7 ou 8, où v vaut 2 à 6.

10. Oligonucléotide de formule I selon l'une ou plusieurs des revendications 7 à 9, où w vaut 2 à 10.

11. Oligonucléotide de formule I selon la revendication 10, où w vaut 2 à 6.

12. Oligonucléotide de formule I selon l'une ou plusieurs des revendications 7 à 11, où z vaut 16 à 23.

13. Oligonucléotide de formule I selon la revendication 12, où z vaut 19 à 21.

14. Oligonucléotide de formule I selon l'une ou plusieurs des revendications 1 à 13, où Z représente adénosine ou 3'-désoxyadénosine.

15. Oligonucléotide de formule I selon l'une ou plusieurs des revendications 1 à 14, dans lequel un ou plusieurs ponts phosphodiester naturels sont remplacés par des ponts internucléotide non naturels qui stabilisent contre une dégradation par une nucléase.

16. Oligonucléotide de formule I selon l'une ou plusieurs des revendications 1 à 15, dans lequel une ou plusieurs liaisons phosphodiester naturelles sont remplacées par des liaisons phosphorothioate.

17. Oligonucléotide de formule I selon l'une ou plusieurs des revendications 1 à 16, dans lequel une ou plusieurs liaisons phosphodiester naturelles sont remplacées par des liaisons phosphorothioate, où ces modifications se trouvent aux extrémités et sur des nucléotides de type pyrimidine internes.

18. Oligonucléotide de formule I selon l'une ou plusieurs des revendications 1 à 5 ou 7 à 17, où Li est un nucléotide naturel ou non naturel.

19. Oligonucléotide de formule I selon l'une ou plusieurs des revendications 1 à 5 ou 7 à 17, où Li est un lieur non nucléotidique.

20. Procédé pour inhiber l'expression génique d'un gène cible dans une cellule in vitro, à l'aide d'un ou de plusieurs oligonucléotides selon l'une ou plusieurs des revendications 1 à 19, où un oligonucléotide complémentaire au gène cible correspondant est d'abord préparé, cet oligonucléotide est introduit dans une cellule, la cellule est incubée, puis l'inhibition de l'expression génique du gène cible est déterminée par des mesures comparatives avec la quantité d'ARNm correspondant ou de produit génique correspondant dans une cellule de contrôle.

21. Procédé selon la revendication 20 pour inhiber l'expression génique d'un gène cible dans une cellule dans laquelle la 2'5'-oligoadénylate-synthase est sous-exprimée ou déficiente par rapport à une cellule de contrôle.

22. Médicament contenant un oligonucléotide selon l'une ou plusieurs des revendications 1 à 19 ainsi que des additifs et/ou des supports et le cas échéant des adjuvants pour la préparation ou la formulation d'un médicament.

23. Utilisation d'un oligonucléotide selon l'une ou plusieurs des revendications 1 à 19 pour l'identification ou la validation de nouveaux gènes cibles thérapeutiques.

24. Utilisation d'un oligonucléotide selon l'une ou plusieurs des revendications 1 à 19 pour l'identification ou la validation de nouveaux gènes cibles thérapeutiques dans la recherche en phytoprotection.

25. Procédé pour la préparation d'un oligonucléotide selon l'une ou plusieurs des revendications 1 à 19, où les oligonucléotides sont d'abord préparés en solution ou sur une phase fixe par couplage successif ou par blocs, le cas échéant hybridés en double brin, puis isolés et purifiés après la préparation.

26. Procédé pour la préparation d'un médicament, où un dérivé oligonucléotidique selon la revendication 25 est préparé et le cas échéant mélangé avec d'autres additifs et/ou supports et le cas échéant des adjuvants.


### SEQUENZPROTOKOLL

<110> Aventis Pharma Deutschland GmbH
<120> Synthetische doppelsträngige Oligonucleotide zur gezielten Hemmung der Genexpression
<130> AVE D-2001/A033
<140>
   <141>
<160> 40
<170> PatentIn Ver. 2.1
<210> 1
   <211> 25
   <212> DNA
   <213> Künstliche Sequenz
<220>
   <223> Beschreibung der künstlichen Sequenz: Bereich aus Photinus pyralis Luziferase
<400> 1
   ttttgaagcg aaggttgtgg atctg 25
<210> 2
   <211> 25
   <212> RNA
   <213> Künstliche Sequenz
<220>
   <223> Beschreibung der künstlichen Sequenz: Bereich aus Photinus pyralis Luziferase
<400> 2
   uuuugaagcg aagguugugg aucug 25
<210> 3
   <211> 80
   <212> DNA
   <213> Künstliche Sequenz
<220>
   <223> Beschreibung der künstlichen Sequenz: Bereich aus Photinus pyralis Luziferase
<400> 3
   gcttttacag atgcacatat cgaggtggac atcacttacg cgaaaatgtc tacgtgtata 60
   gctccacctg tagtgaatgc 80
<210> 4
   <211> 80
   <212> DNA
   <213> Künstliche Sequenz
<220>
   <223> Beschreibung der künstlichen Sequenz: Bereich aus Photinus pyralis Luziferase
<400> 4
   ccgcgaacga catttataat gaacgtgaat tgctcaacag ggcgcttgct gtaaatatta 60
   cttgcactta acgagttgtc 80
<210> 5
   <211> 80
   <212> DNA
   <213> Künstliche Sequenz
<220>
   <223> Beschreibung der künstlichen Sequenz: Bereich aus Photinus pyralis Luziferase
<400> 5
   gcggtcggta aagttgttcc attttttgaa gcgaaggttg cgccagccat ttcaacaagg 60
   taaaaaactt cgcttccaac 80
<210> 6
   <211> 80
   <212> DNA
   <213> Künstliche Sequenz
<220>
   <223> Beschreibung der künstlichen Sequenz: Bereich aus Photinus pyralis Luziferase
<400> 6
   attttttgaa gcgaaggttg tggatctgga taccgggaaa taaaaaactt cgcttccaac 60
   acctagacct atggcccttt 80
<210> 7
   <211> 40
   <212> RNA
   <213> Künstliche Sequenz
<220>
   <223> Beschreibung der künstlichen Sequenz: Bereich aus Photinus pyralis Luziferase
<400> 7
   gcuuuuacag augcacauau cgagguggac aucacuuacg 40
<210> 8
   <211> 40
   <212> RNA
   <213> Künstliche Sequenz
<220>
   <223> Beschreibung der künstlichen Sequenz: Bereich aus Photinus pyralis Luziferase
<400> 8
   cguaagugau guccaccucg auaugugcau cuguaaaagc 40
<210> 9
   <211> 40
   <212> RNA
   <213> Künstliche Sequenz
<220>
   <223> Beschreibung der künstlichen Sequenz: Bereich aus Photinus pyralis Luziferase
<400> 9
   ccgcgaacga cauuuauaau gaacgugaau ugcucaacag 40
<210> 10
   <211> 40
   <212> RNA
   <213> Künstliche Sequenz
<220>
   <223> Beschreibung der künstlichen Sequenz: Bereich aus Photinus pyralis Luziferase
<400> 10
   cuguugagca auucacguuc auuauaaaug ucguucgcgg 40
<210> 11
   <211> 40
   <212> RNA
   <213> Künstliche Sequenz
<220>
   <223> Beschreibung der künstlichen Sequenz: Bereich aus Photinus pyralis Luziferase
<400> 11
   gcggucggua aaguuguucc auuuuuugaa gcgaagguug 40
<210> 12
   <211> 40
   <212> RNA
   <213> Künstliche Sequenz
<220>
   <223> Beschreibung der künstlichen Sequenz: Bereich aus Photinus pyralis Luziferase
<400> 12
   caaccuucgc uucaaaaaau ggaacaacuu uaccgaccgc 40
<210> 13
   <211> 40
   <212> RNA
   <213> Künstliche Sequenz
<220>
   <223> Beschreibung der künstlichen Sequenz: Bereich aus Photinus pyralis Luziferase
<400> 13
   auuuuuugaa gcgaagguug uggaucugga uaccgggaaa 40
<210> 14
   <211> 40
   <212> RNA
   <213> Künstliche Sequenz
<220>
   <223> Beschreibung der künstlichen Sequenz: Bereich aus Photinus pyralis Luziferase
<400> 14
   uuucccggua uccagaucca caaccuucgc uucaaaaaau 40
<210> 15
   <211> 26
   <212> RNA
   <213> Künstliche Sequenz
<220>
   <223> Beschreibung der künstlichen Sequenz: Bereich aus Photinus pyralis Luziferase
<400> 15
   uacagaugca cauaucgagg ugaaaa 26
<210> 16
   <211> 26
   <212> RNA
   <213> Künstliche Sequenz
<220>
   <223> Beschreibung der künstlichen Sequenz: Bereich aus Photinus pyralis Luziferase
<400> 16
   caccucgaua ugugcaucug uaaaaa 26
<210> 17
   <211> 25
   <212> RNA
   <213> Künstliche Sequenz
<220>
   <223> Beschreibung der künstlichen Sequenz: Bereich aus Photinus pyralis Luziferase
<400> 17
   uauaaugaac gugaauugcu caaaa 25
<210> 18
   <211> 25
   <212> RNA
   <213> Künstliche Sequenz
<220>
   <223> Beschreibung der künstlichen Sequenz: Bereich aus Photinus pyralis Luziferase
<400> 18
   gagcaauuca cguucauuau aaaaa 25
<210> 19
   <211> 25
   <212> RNA
   <213> Künstliche Sequenz
<220>
   <223> Beschreibung der künstlichen Sequenz: Bereich aus Photinus pyralis Luziferase
<400> 19
   gguaaaguug uuccauuuuu uaaaa 25
<210> 20
   <211> 25
   <212> RNA
   <213> Künstliche Sequenz
<220>
   <223> Beschreibung der künstlichen Sequenz: Bereich aus Photinus pyralis Luziferase
<400> 20
   cagauccaca accuucgcuu caaaa 25
<210> 21
   <211> 25
   <212> RNA
   <213> Künstliche Sequenz
<220>
   <223> Beschreibung der künstlichen Sequenz: Bereich aus Photinus pyralis Luziferase
<400> 21
   gaagcgaagg uuguggaucu gaaaa 25
<210> 22
   <211> 25
   <212> RNA
   <213> Künstliche Sequenz
<220>
   <223> Beschreibung der künstlichen Sequenz: Bereich aus Photinus pyralis Luziferase
<400> 22
   cagauccaca accuucgcuu caaaa 25
<210> 23
   <211> 21
   <212> RNA
   <213> Künstliche Sequenz
<220>
   <223> Beschreibung der künstlichen Sequenz: Bereich aus Photinus pyralis Luziferase
<400> 23
   gaagcgaagg uuguggaucu g 21
<210> 24
   <211> 27
   <212> RNA
   <213> Künstliche Sequenz
<220>
   <223> Beschreibung der künstlichen Sequenz: Bereich aus Photinus pyralis Luziferase
<400> 24
   cagauccaca accuucgcuu caaaaaa 27
<210> 25
   <211> 60
   <212> RNA
   <213> Künstliche Sequenz
<220>
   <223> Beschreibung der künstlichen Sequenz: Bereich aus humaner EDG1
<400> 25
   gaccucggug guguucauuc ucaucugcug cuuuaucauc cuggagaaca ucuuugucuu 60
<210> 26
   <211> 60
   <212> RNA
   <213> Künstliche Sequenz
<220>
   <223> Beschreibung der künstlichen Sequenz: Bereich aus humaner EDG1
<400> 26
   aagacaaaga uguucuccag gaugauaaag cagcagauga gaaugaacac caccgagguc 60
<210> 27
   <211> 60
   <212> RNA
   <213> Künstliche Sequenz
<220>
   <223> Beschreibung der künstlichen Sequenz: Bereich aus humaner EDG1
<400> 27
   auuuccaagg ccagccgcag cucugagaau guggcgcugc ucaagaccgu aauuaucguc 60
<210> 28
   <211> 60
   <212> RNA
   <213> Künstliche Sequenz
<220>
   <223> Beschreibung der künstlichen Sequenz: Bereich aus humaner EDG1
<400> 28
   gacgauaauu acggucuuga gcagcgccac auucucagag cugcggcugg ccuuggaaau 60
<210> 29
   <211> 21
   <212> RNA
   <213> Künstliche Sequenz
<220>
   <223> Beschreibung der künstlichen Sequenz: Bereich aus humaner EDG1
<400> 29
   aucauccugg agaacaucuu u 21
<210> 30
   <211> 25
   <212> RNA
   <213> Künstliche Sequenz
<220>
   <223> Beschreibung der künstlichen Sequenz: Bereich aus humaner EDG1
<400> 30
   aaagauguuc uccaggauga uaaaa 25
<210> 31
   <211> 21
   <212> RNA
   <213> Künstliche Sequenz
<220>
   <223> Beschreibung der künstlichen Sequenz: Bereich aus humaner EDG1
<400> 31
   ccaaggccag ccgcagcucu g 21
<210> 32
   <211> 25
   <212> RNA
   <213> Künstliche Sequenz
<220>
   <223> Beschreibung der künstlichen Sequenz: Bereich aus humaner EDG1
<400> 32
   cagagcugcg gcuggccuug gaaaa 25
<210> 33
   <211> 21
   <212> RNA
   <213> Künstliche Sequenz
<220>
   <223> Beschreibung der künstlichen Sequenz: Bereich aus humaner EDG1
<400> 33
   ccacggacag acgccgcugu g 21
<210> 34
   <211> 25
   <212> RNA
   <213> Künstliche Sequenz
<220>
   <223> Beschreibung der künstlichen Sequenz: Bereich aus humaner EDG1
<400> 34
   cacagcggcg ucuguccgug gaaaa 25
<210> 35
   <211> 21
   <212> RNA
   <213> Künstliche Sequenz
<220>
   <223> Beschreibung der künstlichen Sequenz: Mutation
<400> 35
   gaagcgaagu ugguggaucu g 21
<210> 36
   <211> 25
   <212> RNA
   <213> Künstliche Sequenz
<220>
   <223> Beschreibung der künstlichen Sequenz: Mutation
<400> 36
   cagauccacc aacuucgcuu caaaa 25
<210> 37
   <211> 21
   <212> RNA
   <213> Künstliche Sequenz
<220>
   <223> Beschreibung der künstlichen Sequenz: Mutation
<400> 37
   gaagagaagu ugguggcucu g 21
<210> 38
   <211> 25
   <212> RNA
   <213> Künstliche Sequenz
<220>
   <223> Beschreibung der künstlichen Sequenz: Mutation
<400> 38
   cagagccacc aacuucucuu caaaa 25
<210> 39
   <211> 21
   <212> RNA
   <213> Künstliche Sequenz
<220>
   <223> Beschreibung der künstlichen Sequenz: Mutation
<400> 39
   cagauccaca accuucgcuu c 21
<210> 40
<211> 21
   <212> RNA
   <213> Künstliche Sequenz
<220>
   <223> Beschreibung der künstlichen Sequenz: Mutation
<400> 40
   cagauccaca accuucgcuu c 21